# EUROPÄISCHE PATENTSCHRIFT

(11) **EP 0 568 816 B1**
(45) Veröffentlichungstag und Bekanntmachung des Hinweises auf die Patenterteilung: **26.06.1996**
(21) Anmeldenummer: 93105425.8
(22) Anmeldetag: 01.04.1993
(51) Int. Cl.: C09B 62/507, C09B 62/09, C07C 317/36, C07C 323/36

(54) **Symmetrische Azofarbstoffe**
Symmetrical azo dyes
Colorants azoiques symétriques

(30) Priorität: 14.04.1992 DE 4212465; 03.06.1992 DE 4218310
(43) Veröffentlichungstag der Anmeldung: 10.11.1993
(73) Patentinhaber: BAYER AG, 51368 Leverkusen (DE)
(72) Erfinder: Herd, Karl-Josef, Dr., W-5068 Odenthal-Holz (DE)

(56) Entgegenhaltungen:
- EP-A- 0 141 996
- EP-A- 0 197 418
- EP-A- 0 279 351
- EP-A- 0 431 389

## Beschreibung

Die vorliegende Erfindung betrifft neue polyfunktionell faserreaktive Mono- beziehungsweise Polyazofarbstoffe.

Poly- und bifunktionelle Mono- und Polyazoreaktivfarbstoffe sind bereits aus einer umfangreichen Literatur bekannt, verwiesen wird insbesondere auf EP-A-197 418, EP-A-51 808, EP-A-279 351, EP-A-141 996, US-A-4 754 024 und US-A-5 107 025. Die bekannten Farbstoffe lassen aber noch Wünsche in bezug auf anwendungstechnische Eigenschaften offen.

Die vorliegende Erfindung betrifft polyfunktionell faserreaktive Mono- beziehungsweise Polyazofarbstoffe, die in Form der freien Säure der Formel (1) entsprechen 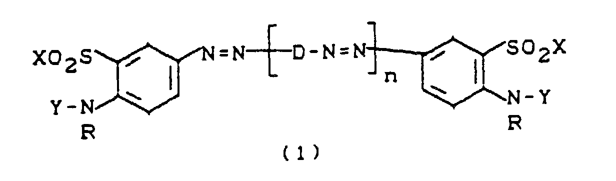 worin
- n =: 0 oder 1,
- X =: CH=CH₂ oder CH₂CF₂OSO₃H,
- Y =: H, C₁-C₆-Alkyl, mit Cl, OH, CN, CO₂H, OSO₃H, SO₃H, SO₂X oder C₁-C₄-Alkoxy substituiertes C₁-C₆-Alkyl, Allyl, Cycloalkyl, wie z.B. Cyclohexyl, Cyclopentyl oder Cyclopropyl, mit CO₂H, OSO₃H oder SO₃H substituiertes Cycloalkyl, Benzyl oder mit OH, CO₂H oder SO₃H substituiertes Benzyl, insbesondere aber CH₂CH₂OSO₃H,
- R =: H, C₁-C₄-Alkyl, mit Cl, OH, CN, CO₂H, OSO₃H, SO₃H, SO₂X oder C₁-C₄-Alkoxy substituiertes C₁-C₄-Alkyl, Allyl, Benzyl oder mit OH, CO₂H bzw. Sulfo substituiertes Benzyl, insbesondere aber H.

Der Substituent Y kann im Rahmen der oben angegebenen Definition jeweils die gleiche oder eine unterschiedliche Bedeutung haben, ebenso der Substituent R.

Die Gruppierung NR-Y steht auch für einen gesättigten N-Heterocyclus, wie z.B. 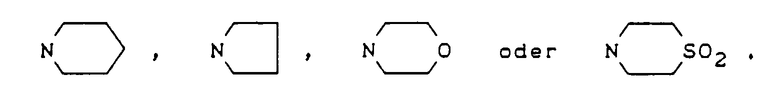
- D =: ein bivalenter aromatischer oder heteroaromatischer Rest der Struktur 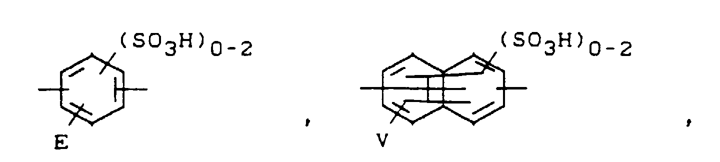 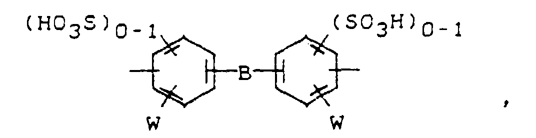 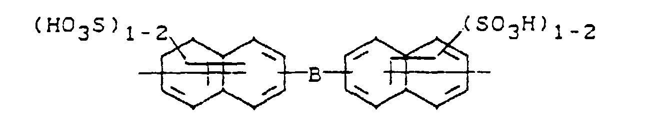 oder 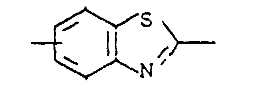
- E, V, W =: unabhängig voneinander Wasserstoff oder C₁-C₄-Alkyl, C₁-C₄-Alkoxy, Halogen oder Carboxy.
- B =: direkte Bindung oder ein Brückenglied, wie z.B. CH₂, CH₂-CH₂, CH=CH, C≡C, O, S, SO, SO₂, CO, CO₂, OCH₂CH₂O, CH₂-O-CH₂, CH₂CH₂OCH₂CH₂, NHCO, NHCONH, NR¹, N=N, N=N→O, O←N=N→O, NHCOCONH, 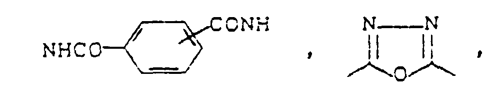 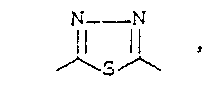 oder ein Triazinylrest der Struktur 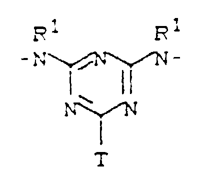 worin
- R¹ =: jeweils unabhängig voneinander H oder C₁-C₄-Alkyl und
- T =: F, Cl, Br, OR², SR², NR³R⁴,
- R² =: H, C₁-C₆-Alkyl, mit OH, Halogen, C₁-C₄-Alkoxy, CO₂H, SO₃H bzw. OSO₃H substituiertes C₁-C₆-Alkyl, Cyclohexyl, Furfuryl, Phenyl, mit OH, CO₂H, SO₃H, C₁-C₄-Alkyl oder C₁-C₄-Alkoxy substituiertes Phenyl oder mit OH, CO₂H oder SO₃H substituiertes oder unsubstituiertes Naphthyl,
- R³, R⁴ =: unabhängig voneinander Wasserstoff, C₁-C₆-Alkyl, C₁-C₄-Alkenyl, mit Halogen, OH, CO₂H, SO₃H, OSO₃H, Methoxy, Ethoxy, SO₂Z oder OCH₂CH₂SO₂Z substituiertes C₁-C₆-Alkyl, Cyclohexyl, Cyclopentyl, Benzyl, Phenyl beziehungsweise Naphthyl sowie mit C₁-C₄-Alkyl, C₁-C₄-Alkoxy, Halogen, CO₂H, SO₃H, SO₂Z oder CH₂SO₂Z substituiertes Phenyl, Benzyl beziehungsweise Naphthyl, wobei R³ und R⁴ zusammen mit dem N-Atom den Rest eines 5- oder 6-gliedrigen Heterocyclus bilden können, wie z.B. 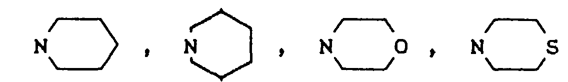 oder 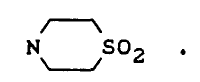
- Z =: CH=CH₂, CH₂CH₂OSO₃H, CH₂CF₂Cl.

Mögliche Substituenten für E, V oder W sind beispielsweise C₁-C₄-Alkyl, C₁-C₄-Alkoxy, Halogen oder Carboxy.

Bevorzugt sind Farbstoffe der Struktur (2) bis (6)

A-N=N-A (2)

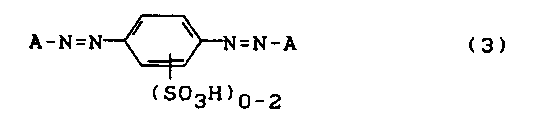

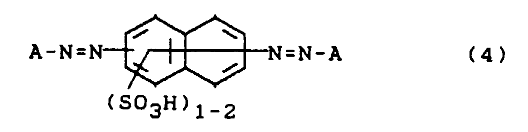

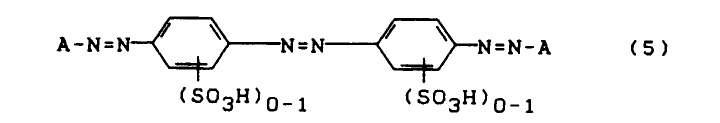

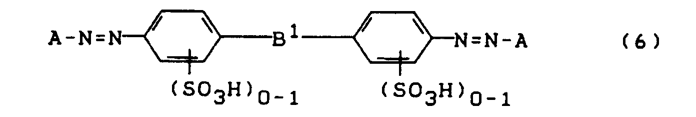

mit 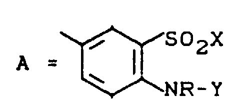 und
- B¹ =: direkte Bindung oder CH₂CH₂, CH=CH, CO, SO₂, NHCONH sowie ein Triazinylrest der Struktur 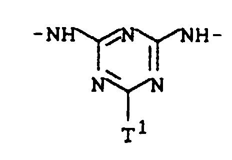 worin
- T¹ =: Cl, F, OH oder NR³R⁴,
- X =: CH=CH₂ oder C₂H₄OSO₃H, wobei

R, Y, R³ und R⁴ die obengenannte Bedeutung haben.

Besonders bevorzugt sind folgende neue Reaktivfarbstoffe der Struktur (7) bis (14) 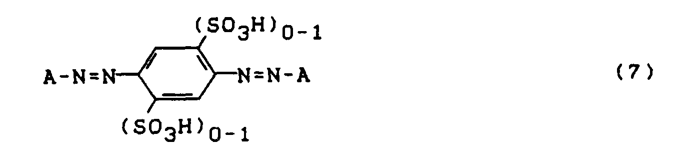 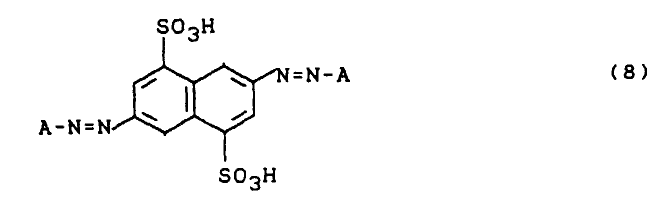 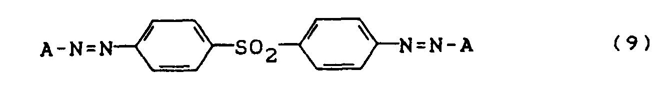 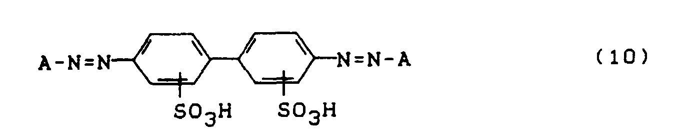 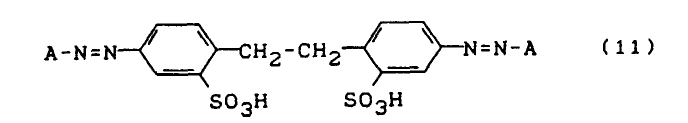 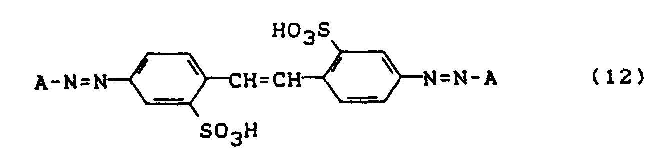 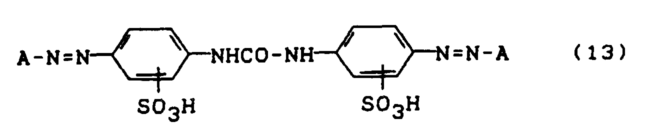 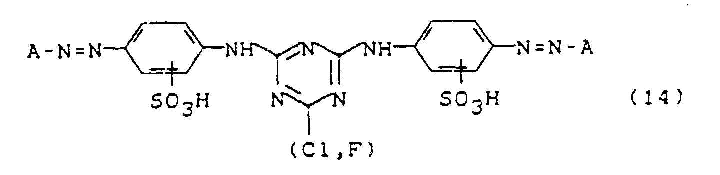 worin A die obige Bedeutung hat, insbesondere aber 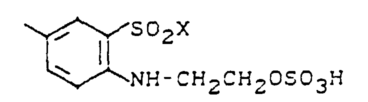 bedeutet.

Die Herstellung der Farbstoffe kann nach verschiedenen Methoden, erfolgen:
1. Sulfatierung der nicht faserreaktiven hydroxyethylgruppenhaltigen Zwischenprodukte der Formel (15) zu den faserreaktiven Farbstoffe der Formel (1) mit X = CH₂CH₂OSO₃H und gegebenenfalls Überführung unter schwach alkalischen Bedingungen in solche mit X = CH=CH₂. 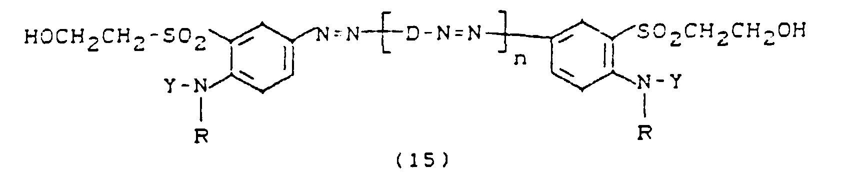
   Hydroxyalkylgruppen in R und Y werden dabei ebenfalls in Sulfatoalkylgruppen überführt.
   Die Synthese von (15) mit n=O, R=H und Y=CH₂CH₂OH ist z.B. in DE-A 3 939 966 beschrieben.
   Farbstoffzwischenprodukte der Struktur (15) mit n=1 sind herstellbar
   a) durch Tetrazotieren von Diaminoverbindungen H₂N-D-NH₂, Kupplung auf zwei Äquivalente der Komponente (16) 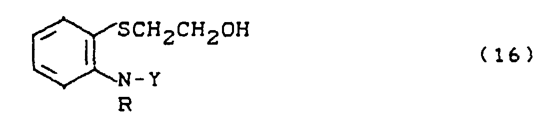
      und Oxidation der resultierenden Bismercaptoverbindungen der Struktur (17), 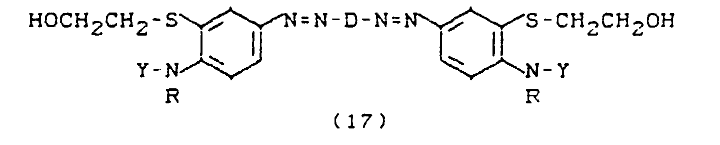
   b) durch Diazotieren von Aminoazo-Zwischenprodukten der Struktur (18) beziehungsweise (19), 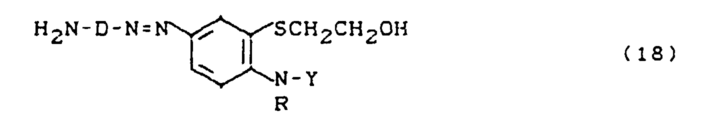 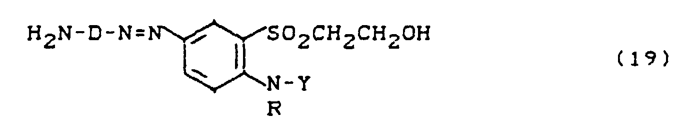
      Kupplung der Diazoniumsalze auf (16) und nachfolgende Oxidation der resultierenden Mercaptoverbindungen (17) beziehungsweise (20), 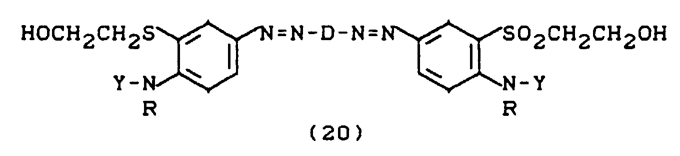
   c) durch Kondensation von zwei Äquivalenten der Verbindungen (19) mit Kohlensäurederivaten, wie z.B. Phosgen, mit Oxalylchlorid, Terephthaloylchlorid oder mit Triazinen der Formel (21) 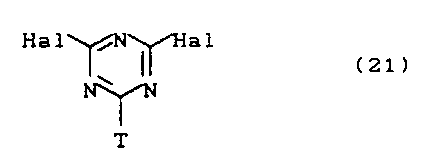 worin Hal = Cl, Br, F
      und T die Bedeutung wie unter Formel (1) zukommt,
      beziehungsweise mit Triazinen der Formel (24) 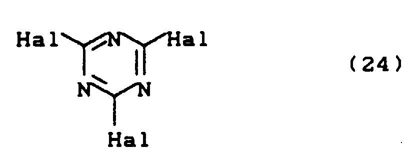 wobei im letzten Fall eine Kondensation mit HOR², HSR² oder HNR³R⁴ folgen kann,
   d) durch Kondensation von 2 Äquivalenten der Verbindungen (18) mit Kohlensäurederivaten, wie z.B. Phosgen, mit Oxalylchlorid oder Terephthaloylchlorid zu Verbindungen der Formel (17) und nachfolgende Oxidation.
      Zwischenprodukte (18) erhält man durch Diazotieren von O₂N-D-NH₂ beziehungsweise Acyl-NH-D-NH₂, Kupplung des Diazoniumsalzes auf (16) und nachfolgende Reduktion der Nitrogruppe in (25) beziehungsweise Hydrolyse der Acylaminogruppe in (26), 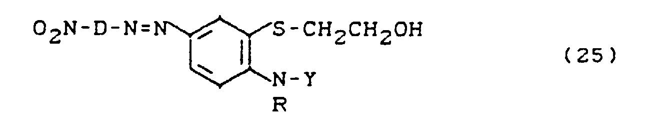 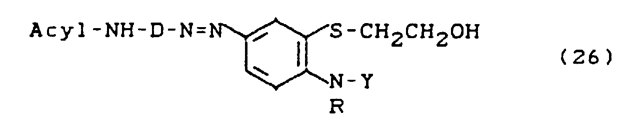 worin Acyl bevorzugt die Bedeutung von Acetyl, Oxalyl, Benzoyl und Formyl zukommt.
      Zwischenprodukte (19) erhält man z.B. durch direkte Oxidation von Azosulfiden der Struktur (18) oder aber durch Diazotieren von O₂N-D-NH₂ beziehungsweise Acyl-NH-D-NH₂, Kupplung der Diazoniumsalze auf (16), Oxidation zu Sulfonen der Struktur (27) beziehungsweise (28) und abschließende Reduktion der Nitrogruppe in (27) beziehungsweise Hydrolyse der Acylaminogruppe in (28). 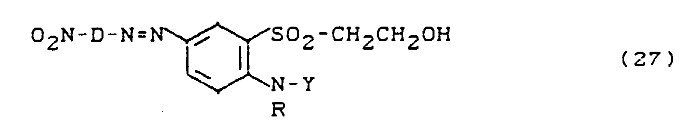 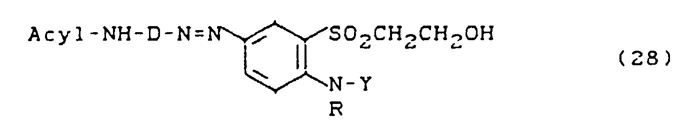
      Trisazoverbindungen der Struktur (15) beziehungsweise (17), in denen das Brückenglied B in D für eine Azo- beziehungsweise Azoxy-Gruppierung steht, erhält man z.B. durch Zusammenreduktion von Nitroverbindungen (25) beziehungsweise (27), in denen der Rest D keine Brückenglieder B enthält.
2. Diazotieren von Aminoverbindungen der Struktur (29) beziehungsweise (30), 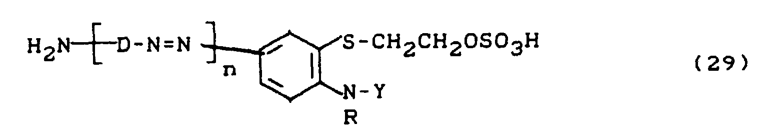 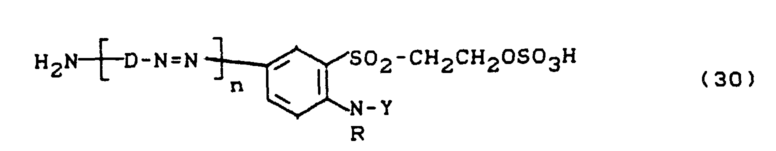 die durch Sulfatierung von (18) beziehungsweise (19) oder (31) 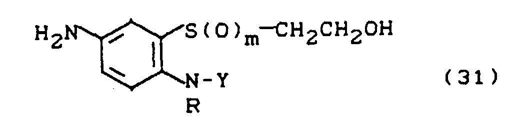 m = 0 oder 2 zugänglich sind, Kupplung der Diazoniumsalze auf (16) und Oxidation und Sulfatierung der Zwischenstufen der Struktur (32) beziehungsweise (33). 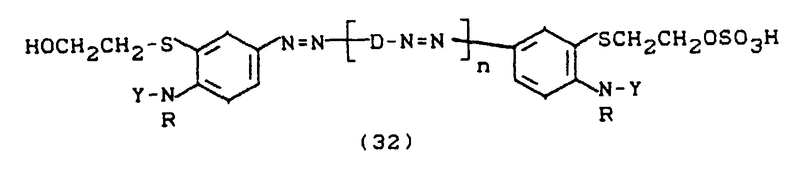 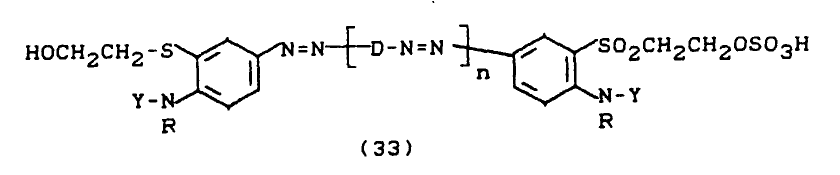 Sulfatiert werden dabei auch Hydroxyalkylgruppen in R oder Y.
3. Kondensation von 2 Äquivalenten der Aminoazoverbindungen (30) mit n=1 mit Kohlensäurederivaten, wie z.B. Phosgen, mit Oxalylchlorid, Terephthaloylchlorid oder mit Triazinen (21) sowie mit Triazinen (24), wobei im letzten Fall eine Kondensation mit HOR², HSR² oder HNR³R⁴ erfolgen kann.
4. Kondensation von 2 Äquivalenten der Aminoazoverbindungen (29) mit n=1 mit Kohlensäurederivaten, wie z.B. Phosgen, mit Oxalylchlorid oder Terephthaloychlorid und Oxidation der resultierenden Bis-mercaptoverbindungen (34). 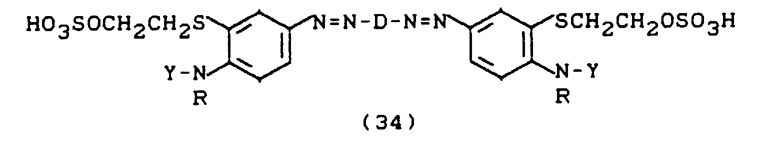
   (34) ist auch durch Sulfatierung von entsprechenden Derivaten der Struktur (17) zugänglich. Hydroxyalkylgruppen in den Substituenten R und Y werden dabei ebenfalls in Sulfatoalkylgruppen überführt.
   Die Sulfatierungsreaktionen werden bevorzugt mit konzentrierter Schwefelsäure, Schwefelsäure-monohydrat, Oleum oder Schwefeltrioxid durchgeführt. Für die Oxidation der Sulfide zu den Sulfonen setzt man bevorzugt Wasserstoffperoxid unter Wolframat-Katalyse ein und setzt in wässrigem Medium um. Die Zusammenreduktion von Nitroverbindungen der Struktur (25) und (27) zu Trisazo/azoxy-Derivaten nimmt man am vorteilhaftesten mit schonenden Reduktionsmitteln, wie z.B. Traubenzucker, Maltose oder Saccharose, vor. Zur Reduktion von Nitroazoverbindungen zu den entsprechenden Aminoazoverbindungen verwendet man bevorzugt Natriumsulfid oder Natriumhydrogensulfid. Diazotierungs-, Tetrazotierungs- und Azokupplungsreaktionen erfolgen nach literaturbekannten Verfahren.

Die Kupplungskomponenten (16) sind durch Ethoxylierung von o-Aminothiophenol und N-substituierten o-Aminothiophenolen mit Ethylenoxid oder Chlorethanol unter alkalischen Bedingungen herstellbar. Die Komponente (16) mit R = H und Y = CH₂CH₂OH ist durch Umsetzung von Benzthiazol, Ethylenoxid und Wasser und nachfolgende Hydrolyse der resultierenden N-Formylverbindung zugänglich (vgl. DE-A 3 939 966)

Eine andere Möglichkeit zur Herstellung von Verbindungen (16) besteht in der Alkylierung von Vorstufen der Formel 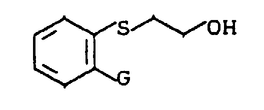 worin G = NH₂, NHR oder NHY.

Die Alkylierung kann z.B. mit Alkylhalogeniden R-Hal bzw. Y-Hal, worin Hal = Cl, Br, F, mit Alkylsulfaten bzw. Dialkylsulfaten, mit aktivierten Olefinen, wie z.B. Acrylsäurederivaten oder Vinylsulfonylderivaten, oder reduktiv mit Aldehydderivaten erfolgen.

Bevorzugt sind solche Kupplungskomponenten (16), bei denen die Reste R und Y mit einer Sulfo, Carboxy oder insbesondere mit einer Hydroxygruppe substituiert sind, Beispiele für (16) sind:

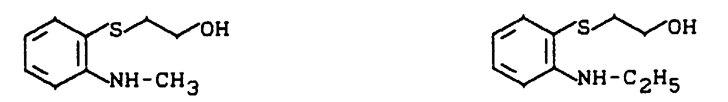

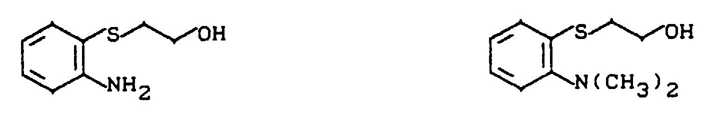

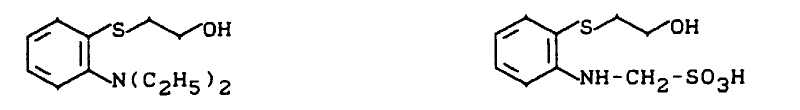

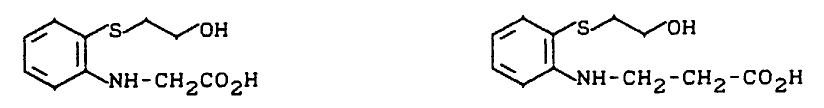

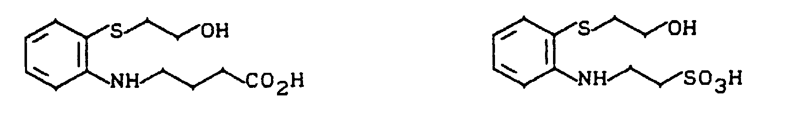

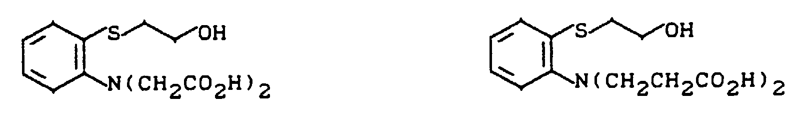

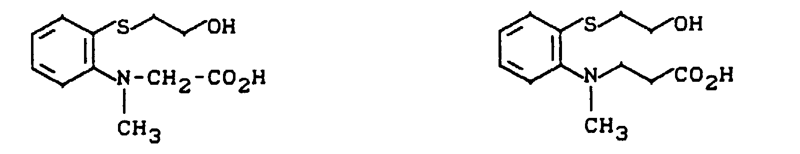

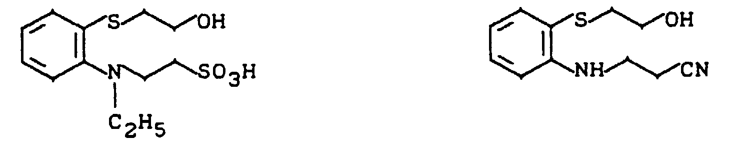

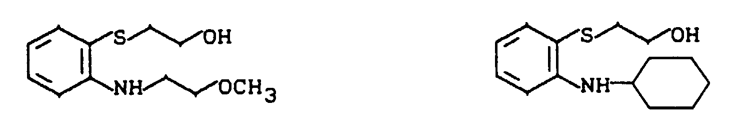

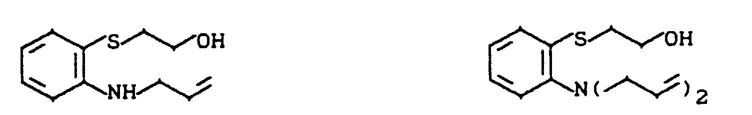

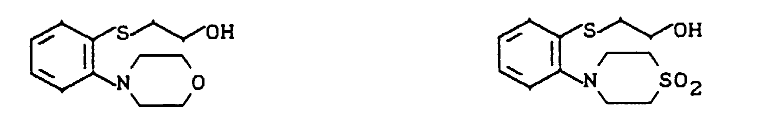

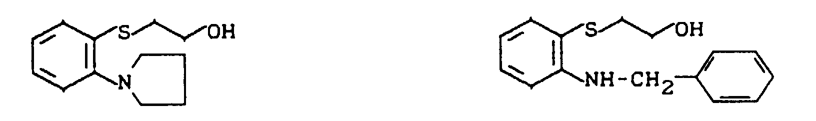

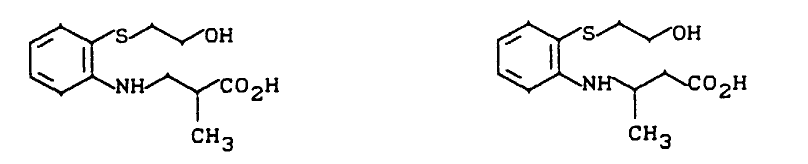

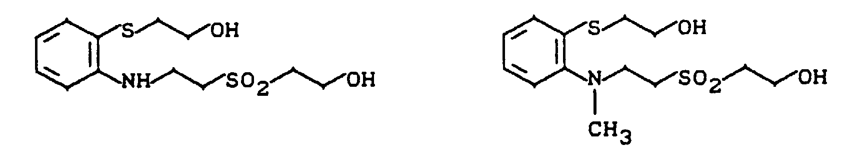

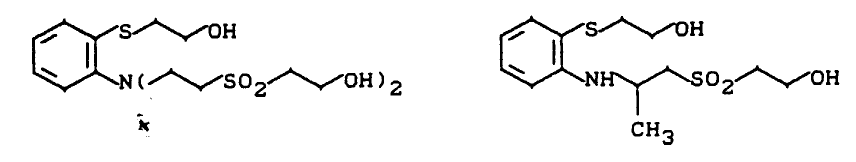

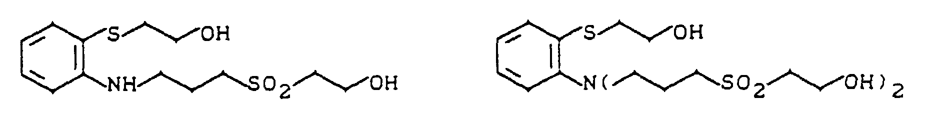

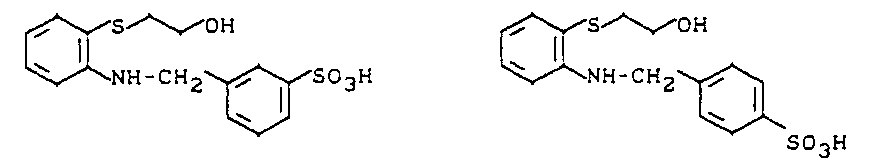

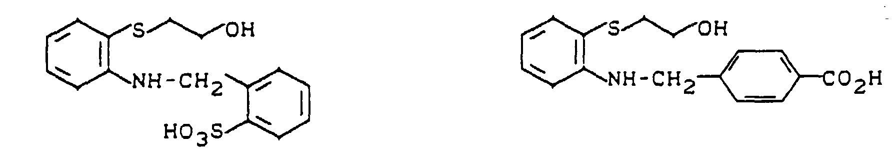

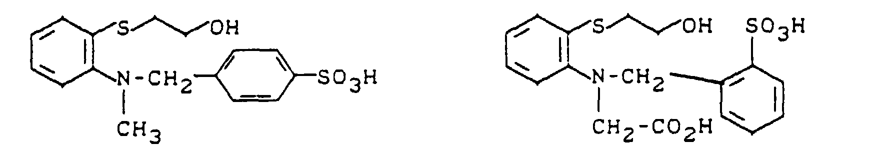

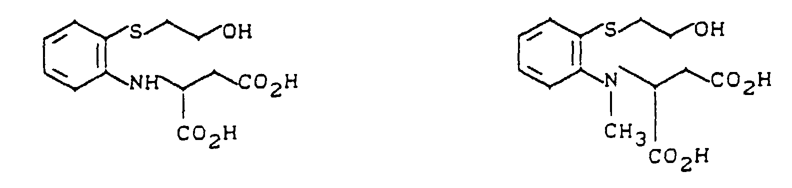

Gegenstand der Erfindunq sind auch die Zwischenkomponenten folgender allgemeiner Formeln: 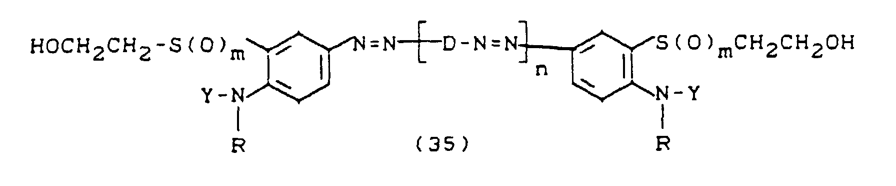 worin
m = unabhängig voneinander 0 oder 2,
n = 1 und
R, Y und D die unter Formel (1) genannte Bedeutung zukommt. 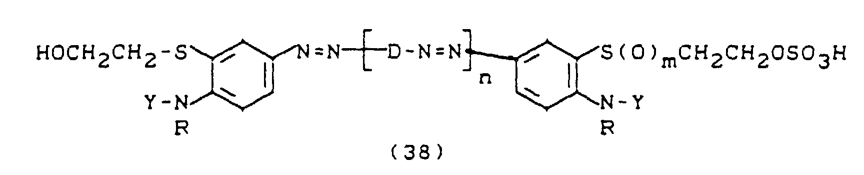
worin
m = 0 oder 2.
n = 0 oder 1 und
R, Y und D die unter Formel (1) angegebene Bedeutung haben. 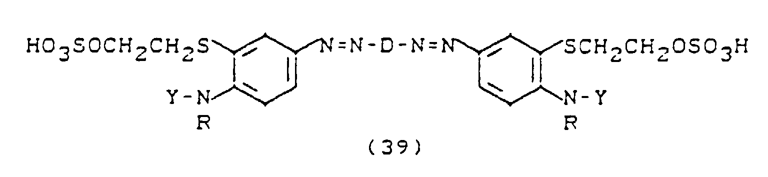
worin
R, Y und D die unter Formel (1) genannte Bedeutung zukommt.

Die angegebenen Formeln sind die der freien Säuren. Bei der Herstellung werden im allgemeinen die Salze erhalten, insbesondere die Alkalisalze, wie Natrium-, Kalium- oder Lithiumsalze.

Die neuen erfindungsgemäßen Farbstoffe eignen sich zum Färben und Bedrucken von hydroxyl- und amidgruppenhaltigen Materialien, insbesondere Wolle und Cellulosematerialien. Sie zeichnen sich durch eine hohe Reaktivität und einen hohen Fixiergrad aus. Die mit diesen Farbstoffen erhältlichen gelben, orangen und braunen Färbungen und Drucke zeichnen sich außerdem durch eine hohe Faser-Farbstoff-Bindungsstabilität sowie durch eine hervorragende Stabilität gegenüber Oxidationsmitteln, wie peroxid- oder chlorhaltigen Waschmitteln, aus.

Die Farbstoffe können sowohl als Feststofformierungen als auch als konzentrierte Lösungen eingesetzt werden.

Sie eignen sich auch in Mischungen mit anderen Reaktivfarbstoffen, insbesondere für Trichromien`

Die Herstellung und Verwendung soll anhand der nachfolgenden Beispiele erläutert werden.

### Beispiel 1

51,2 g der Azoverbindung der Formel 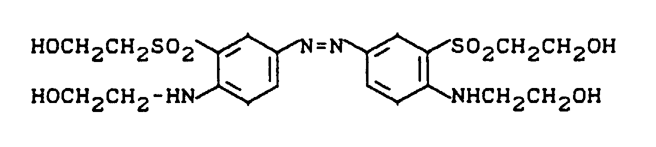 werden in 250 ml Schwefelsäure-monohydrat bei 20 bis 25°C eingetragen und bis zur Lösung verrührt. Anschließend gießt man vorsichtig auf eine Vorlage aus 200 ml Wasser und 400 g Eis. Zur Ausfällung des Farbstoffs als Tetranatriumsalz fügt man bei 0 bis 10°C portionsweise ca. 12 g feste wasserfreie Soda. Zur Vervollständigung der Fällung kann auch Kochsalz nachgesetzt werden. Der orangefarbene Niederschlag wird durch Abnutschen isoliert, in 200 ml Wasser erneut angerührt und mit Sodalösung neutralisiert (pH 6). Nach Sprühtrocknung resultieren ca. 90 g eines Farbstoffpulvers, dem die Struktur 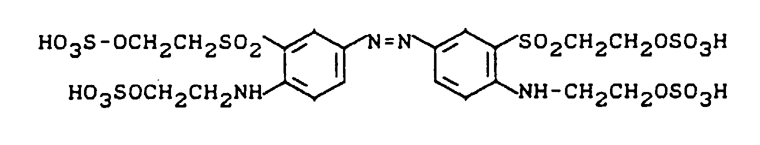 zukommt und das Baumwolle in klaren gelben Farbtönen färbt (λₘₐₓ = 410, 436 nm (H₂O)).

### Beispiel 2

54 g 2,2'-(1,2-Ethandiyl)bis[5-aminobenzolsulfonsäure] werden in 300 ml Wasser, 100 g Eis und 60 ml konz. Salzsäure verrührt und danach durch Zugabe von 70 ml einer 30 Vol%igen wäßrigen Natriumnitritlösung tetrazotiert. Die Suspension wird 1 Stunde bei 5°C gerührt. Der Nitritüberschuß wird durch Zugabe von Amidosulfonsäure nach Reaktionsende entfernt. Dazu fügt man nun eine Lösung 65 g 2-(2-Hydroxyethylmercapto)-N-(2-hydroxyethyl)anilin in 50 ml Wasser/15 ml konz. Salzsäure. Nach 30 Minuten stellt man bei ca. 10°C den pH-Wert mit Sodalösung innerhalb einer Stunde auf pH 3,0 bis 3,5 und läßt vier Stunden rühren. Das ausgefallene Zwischenprodukt der Struktur 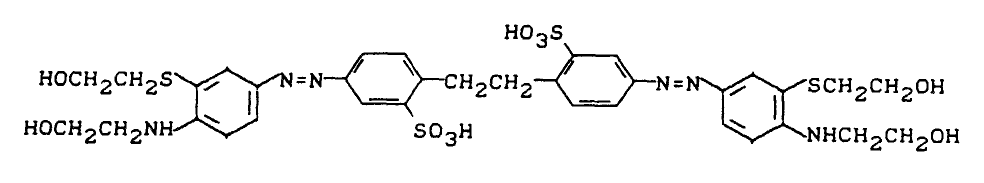 (λₘₐₓ = 440 nm) wird durch Abnutschen isoliert, und die feuchte Paste wird direkt weiter umgesetzt. Es kann aber auch auf eine Zwischenisolierung verzichtet werden und die Kupplungsmischung direkt oxidiert werden.

Zur Oxidation suspendiert man die feuchte Paste in 400 ml Wasser, stellt mit Sodalösung auf pH 8,5 und fügt 0,2 g Natriumwolframat zu. Es wird auf 60°C erwärmt. Ohne weitere Wärmezufuhr dosiert man nun langsam ca. 100 ml einer 30%igen wässrigen Wasserstoffperoxidlösung zu, wobei die Temperatur aufgrund der Reaktionswärme auf ca. 70 bis 75°C ansteigt, und eine Lösung resultiert. Nach einer einstündigen Nachrührphase bei 75°C kühlt man auf 20°C ab, isoliert den ausgefallenen Farbstoff nach Aussalzen und trocknet. Es werden ca. 90 g salzhaltiges Produkt der Struktur

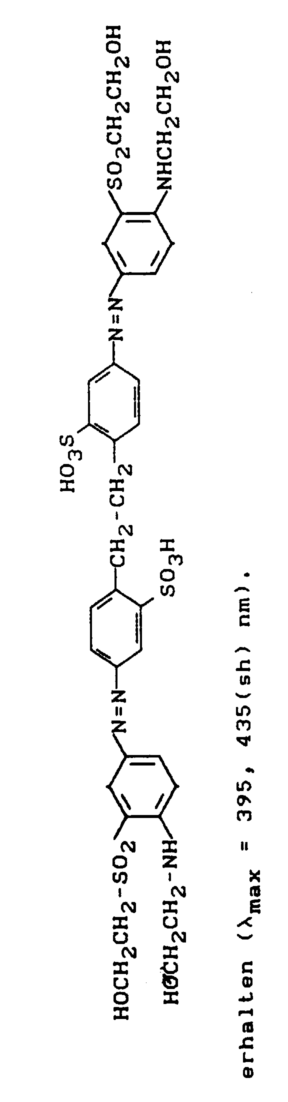

Diese Menge wird portionsweise bei 20-25°C in 250 ml Schwefelsäure-monohydrat eingetragen und für ca. 3 Stunden zur Lösung verrührt. Beim Austragen auf 200 ml Wasser/400 g Eis fällt der Farbstoff kristallin aus. Es werden portionsweise 20 g feste Soda eingetragen, und danach wird abgesaugt. Die Isolierung wird in 200 ml Wasser angerührt und mit Sodalösung neutralisiert. Nach Sprühtrocknung dieser Mischung werden ca. 150 g salzhaltiger Reaktivfarbstoff erhalten, dem die Struktur 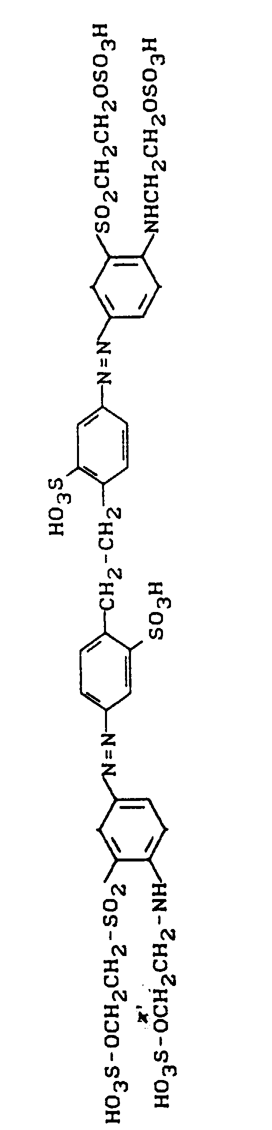 zukommt und der Baumwolle in gelben Farbtönen färbt (λₘₐₓ = 389, 435(sh) nm).

Durch Variation der Tetrazokomponente in Beispiel 2 werden weitere interessante und wichtige Disazofarbstoffe beziehungsweise Trisazofarbstoffe erhalten:

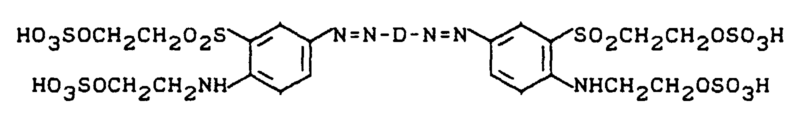

### Beispiel 3:

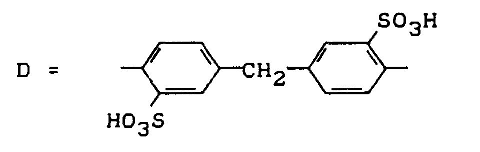

### Beispiel 4:

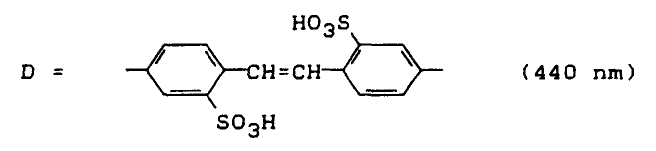

### Beispiel 5:

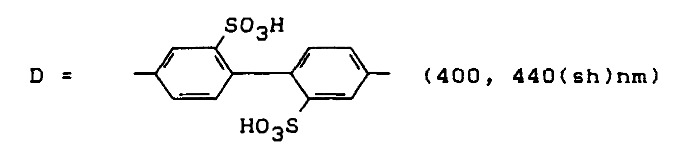

### Beispiel 6:

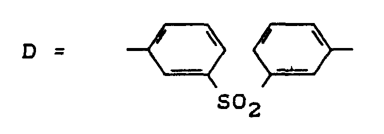

### Beispiel 7:

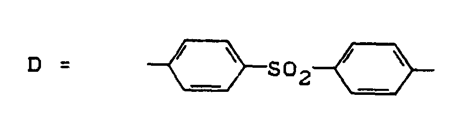

### Beispiel 8:

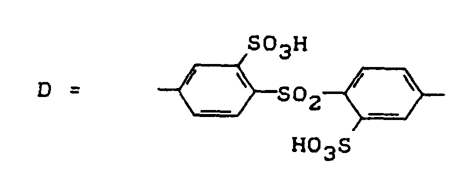

### Beispiel 9:

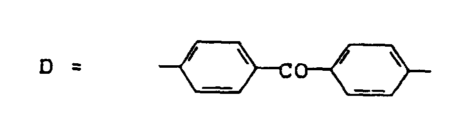

### Beispiel 10:

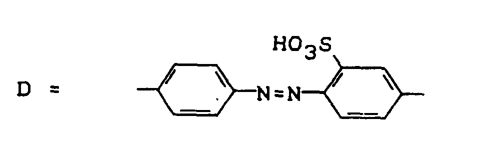

### Beispiel 11:

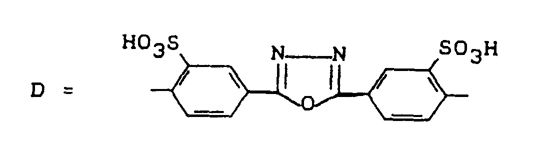

### Beispiel 12:

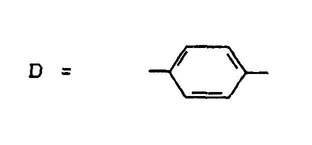

### Beispiel 13:

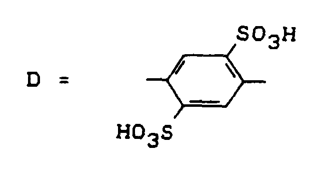

### Beispiel 14

31,6 g 2-Amino-5-nitrobenzolsulfonsäure werden in 250 ml Wasser und 40 ml konz. Salzsäure bei 70°C gelöst. Durch Einwerfen von Eis wird auf 15°C abgekühlt und mit 35 ml 30 Vol%iger wäßriger Natriumnitritlösung diazotiert. Nach 30 Minuten Nachrührzeit bei 15°C wird der Nitritüberschuß mit Amidosulfonsäure entfernt. Dazu fügt man 32,5 g 2-(2-Hydroxyethylmercapto)-N-(2-hydroxyethyl)anilin und stellt innerhalb von 3 Stunden den pH-Wert der Mischung langsam auf 4,5. Es wird mit 30 g Kochsalz ausgesalzen, die Ausfällung isoliert und die erhaltene Paste erneut in 250 ml Wasser angerührt. Nach Zusatz von 0,2 g Natriumwolframat wird auf 70°C erwärmt und bei pH 8,5 bis 9,0 mit 60 ml 30%iger Wasserstoffperoxidlösung oxidiert` Die Temperatur steigt dabei auf 80 bis 85°C an. Nach dem Abkühlen wird das ausgefallene Zwischen-produkt der Struktur (λₘₐₓ = 400 nm) 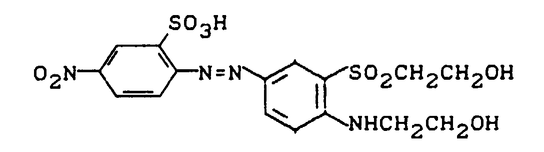 durch Abnutschen isoliert.

Diese 130 g feuchte Nitroazoverbindung werden in 300 ml Wasser und bei pH 7 auf 80°C erwärmt. Es werden anschließend 50 ml einer ca. 30%igen wäßrigen Natriumhydrogensulfidlösung innerhalb von 20 Minuten zudosiert und 2 Stunden bei 80°C gerührt. Das Ende der Reduktion wird dünnschichtchromatographisch überprüft. Danach versetzt man mit 5 g Aktivkohle und klärt die heiße Lösung ab. Nach dem Abkühlen auf 20°C stellt man auf pH 5,0 und salzt mit 40 g Kaliumchlorid aus. Nach Absaugen und Trocknen erhält man 70 g salzhaltiges Aminoazo-Zwischenprodukt der Struktur 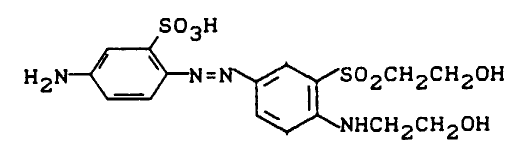 (λₘₐₓ = 404, 445(sh) nm).

### Beispiel 15

70 g des Zwischenproduktes von Beispiel 14 werden in 400 ml Wasser, 100 g Eis und 40 ml konz. Salzsäure verrührt und durch Zudosieren von ca. 25 ml der 30 Vol%igen Natriumnitritlösung bei 5 bis 15°C diazotiert. Nach einer zweistündigen Nachrührphase werden 27 g 2-(2-Hydroxyethylmercapto)-N-(2-hydroxyethyl)anilin zugefügt und der pH-Wert mit Sodalösung innerhalb von 3 Stunden langsam auf 5,0 erhöht (Temperatur: ca. 20°C). Nach Beendigung der Kupplungsreaktion versetzt man mit 0,3 g Natriumwolframat, erwärmt die Mischung auf 70°C, stellt auf pH 8,0 und dosiert so 50 ml Wasserstoffperoxidlösung zu, daß die Temperatur 80°C nicht übersteigt. Der Fortgang der Reaktion wird mittels DC verfolgt. Sind nur noch Spuren Sulfoxid-Verbindung nachweisbar, wird abgekühlt, wobei das Disazodisulfon auskristallisiert` Isolierung und Trocknung liefern ca. 75 g der Reaktivfarbstoffvorstufe der Struktur

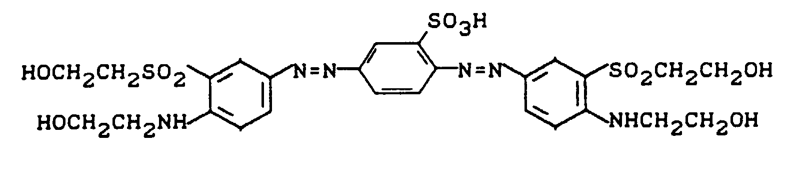

### Beispiel 16

Diese Vorstufe aus Beispiel 15 erhält man auch, wenn man die Zwischenverbindung der Struktur
(λₘₐₓ = 400, 430(sh) nm) 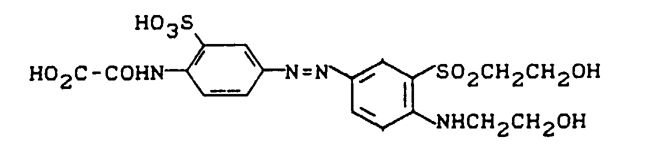 die durch Diazotieren von N-(4-Amino-2-sulfophenyl)oxalsäuremonoamid, kuppeln auf 2-(2-Hydroxyethylmercapto)-N-(2-hydroxyethyl)anilin und H₂O₂-Oxidation erhalten wird, sauer verseift, die resultierende Aminoverbindung diazotiert, auf 2-(2-Hydroxyethylmercapto)-N-(2-hydroxyethyl)anilin kuppelt und mit Wasserstoffperoxid oxidiert.

### Beispiel 17

Zur gleichen Reaktivfarbstoffvorstufe aus Beispiel 15 gelangt man auch folgendermaßen:

Man nimmt den Azofarbstoff der ersten Kupplung aus Beispiel 14, dem die Struktur (λₘₐₓ = 383, 453 nm (H₂O)) 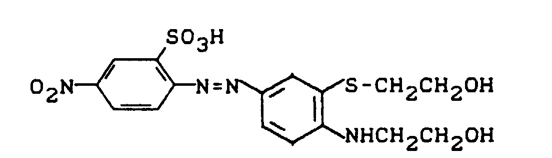 zukommt und der als feuchte Paste anfällt, in 250 ml Wasser auf und erwärmt bei pH 7 auf 80°C. Dazu fügt man ca. 50 ml einer 30%igen wäßrigen Natriumhydrogensulfidlösung und rührt 2 Stunden bei 80°C. Nach Reduktionsende (DC-Kontrolle) und Aktivkohlezusatz wird heiß geklärt. Die abgekühlte Lösung wird auf pH 1 gestellt, und die resultierende Suspension mit ca. 30 ml einer 30 Vol%igen Natriumnitritlösung bei 5°C diazotiert. Das Diazoniumsalz wird, wie bereits oben beschrieben, auf die Anilinkupplungskomponente gekuppelt. Es resultiert eine Verbindung der Struktur 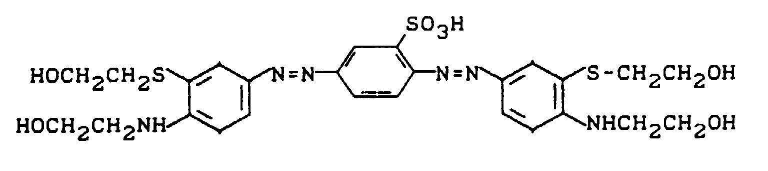 die zwei Mercaptoreste aufweist und die aus der Kupplungsmischung durch Absaugen isoliert wird, Diese wird in Analogie zur Vorschrift in Beispiel 15 mittels Wasserstoffperoxidlösung bei 80°C und pH 8,0 oxidiert, Ein deutlich erhöhter Verbrauch an Oxidationsmittel ist allerdings erforderlich.

Isolierung und Trocknung liefert die in Beispiel 15 genannte Reaktivfarbstoffvorstufe.

### Beispiel 18

75 g des Disazodisulfons aus Beispiel 15 werden in 200 ml Schwefelsäure-monohydrat bei 20 bis 25°C portionsweise eingetragen und bis zur Lösung bei 25 bis 30°C verrührt. Beim Austragen auf 200 ml Wasser/400 g Eis fällt der Farbstoff bereits teilweise kristallin aus. Eine vollständigere Fällung erreicht man nach Zudosieren von 30 bis 40 g fester Soda. Es wird abgesaugt, und die isolierte Paste in 250 ml Wasser angerührt. Nach Neutralisation mittels Sodalösung auf pH 6,0 wird die Mischung sprühgetrocknet, beziehungsweise an Rotationsverdampfer bis zur Trockene aufkonzentriert. Man erhält ca. 130 g salzhaltigen Reaktivfarbstoff der Struktur 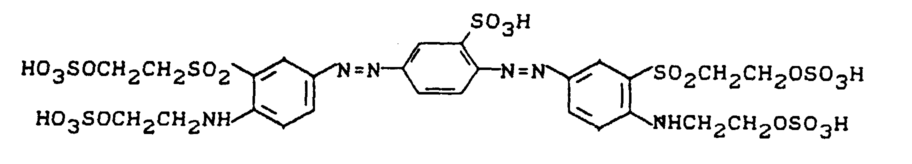 der Baumwolle in klaren goldgelben Nuancen färbt.

Eine andere Variante der Herstellung von Beispiel 18 ist die Umsetzung des Disazodisulfons aus Beispiel 15 mit Oleum im Laborkneter. Dabei wird die Reaktionsmischung bei ca. 70 bis 80°C/180 mbar eindosiert und dann die Temperatur auf ca. 140°C/2 mbar erhöht. Es wird zur Trockene aufkonzentriert, wobei ca. 115 g Farbstoffpulver anfallen.

### Beispiel 19

Setzt man als Startkomponente in Beispiel 14 anstelle von 2-Amino-5-nitrobenzolsulfonsäure nun 2-Amino-6-nitro-4,8-naphthalindisulfonsäure ein und verfährt in Analogie zu Beispiel 14, 15 oder 17 und 18, so resultiert ein goldgelber Reaktivfarbstoff der Struktur

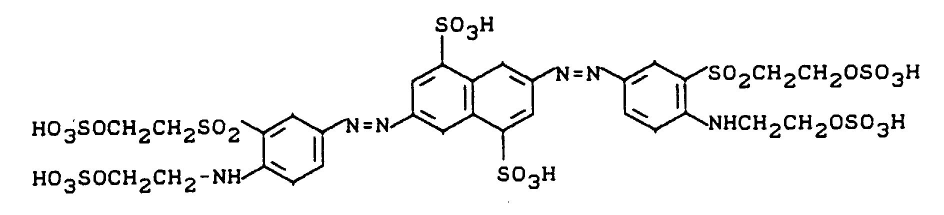

### Beispiel 20

70 g des Aminoazozwischenprodukts aus Beispiel 14 werden in 200 ml Schwefelsäure bei 20 bis 25°C eingetragen und für 2 Stunden bei 30°C gerührt. Die Lösung wird vorsichtig auf eine Vorlage aus 200 ml Wasser/400 g Eis ausgetragen. Zur Ausfällung des Dinatriumsalzes wird portionsweise mit 20 g fester Soda versetzt und gegebenenfalls mit diversen Mengen an Kochsalz ausgesalzen. Der orangegelbe Niederschlag wird isoliert und bei pH 6,0 in 300 ml Wasser gelöst. Das faserreaktive Zwischenprodukt der Struktur (λₘₐₓ = 403, 440(sh) nm) 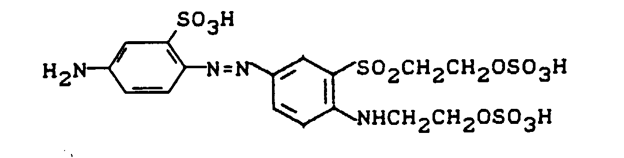 wird am günstigsten in Lösung weiter umgesetzt.

### Beispiel 20 A

In die Lösung des Zwischenprodukts aus Beispiel 19 wird unter entsprechenden Sicherheitsvorkehrungen bei 30°C und pH 6 bis 6,5 solange Phosgen eingeleitet, bis keine freie Farbbase mehr nachweisbar ist.

Der pH-Wert wird durch gleichmäßige Zugabe von Sodalösung konstant gehalten. Nach Reaktionsende wird zur Vernichtung restlichen Phosgens 2 Stunden bei 40°C nachgerührt (pH 6,0). Es wird auf 20°C abgekühlt und mit 30 g Kochsalz ausgesalzen. Isolierung und Trocknung liefern ca. 125 g eines orangegelben salzhaltigen Farbstoffpulvers, das die Struktur 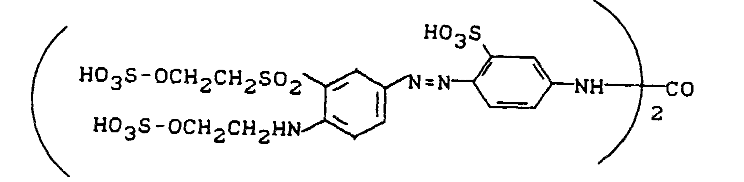 aufweist und Baumwolle in gelben Farbtönen färbt.

### Beispiel 20 B

Die Lösung aus Beispiel 19 wird bei 10 bis 15°C und konstantem pH 6,0 bis 6,5 mit 13 g Cyanurchlorid umgesetzt. Der pH-Wert wird mit Sodalösung kontrolliert. Nach ca. 2 Stunden ist die Reaktion beendet. Es wird nochmal eine Stunde bei 30°C gerührt, die Lösung mittels Filterhilfe geklärt und der Farbstoff durch Zugabe ausreichender Mengen Kochsalz ausgesalzen. Es werden ca. 135 g salzhaltiger Reaktivfarbstoff isoliert, dem die Struktur 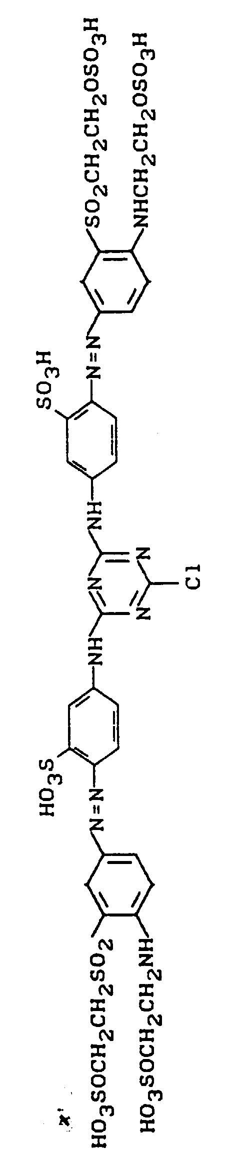 zukommt und der Baumwolle in gelben Farbtönen färbt.

Wählt man anstelle von Cyanurchlorid in Beispiel 20 B äquimolare Mengen an Cyanurfluorid, 2-(2-Sulfoethyl)amino-4,6-dichlortriazin, 2-(3-Sulfophenyl)amino-4,6-difluortriazin oder andere vergleichbare Dihalogentriazinderivate, so gelangt man zu folgenden interessanten Gelbfarbstoffen:

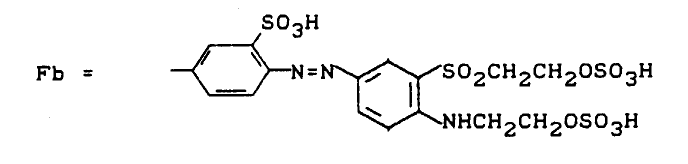

### Beispiel 21:

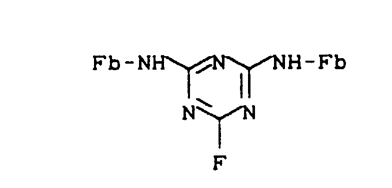

### Beispiel 22:

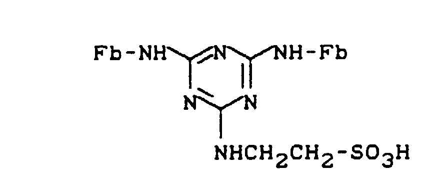

### Bespiel 23:

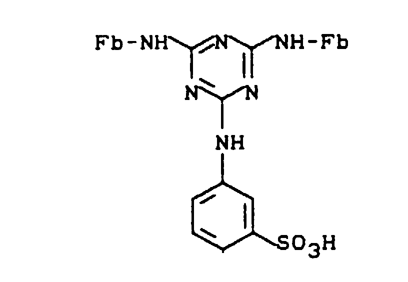

### Beispiel 24:

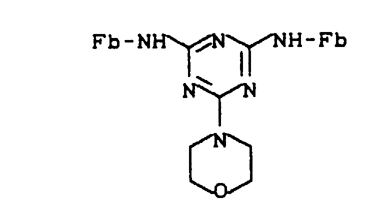

### Beispiel 25:

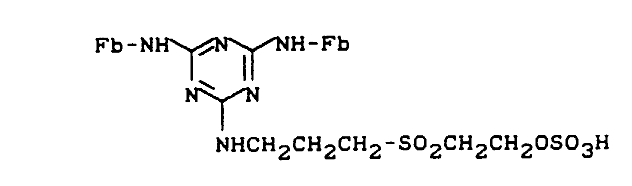

### Beispiel 26:

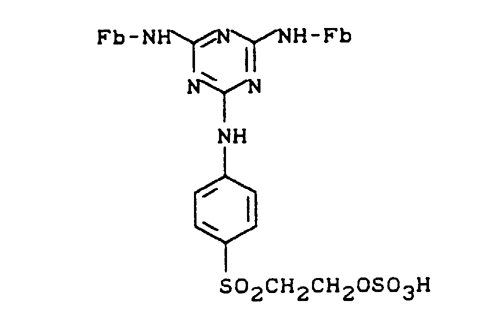

Durch Variation der Kupplungskomponente in den vorausgegangenen Beispielen sind in Analogie folgende ebenfalls wichtige Reaktivfarbstoffbeispiele zugänglich:

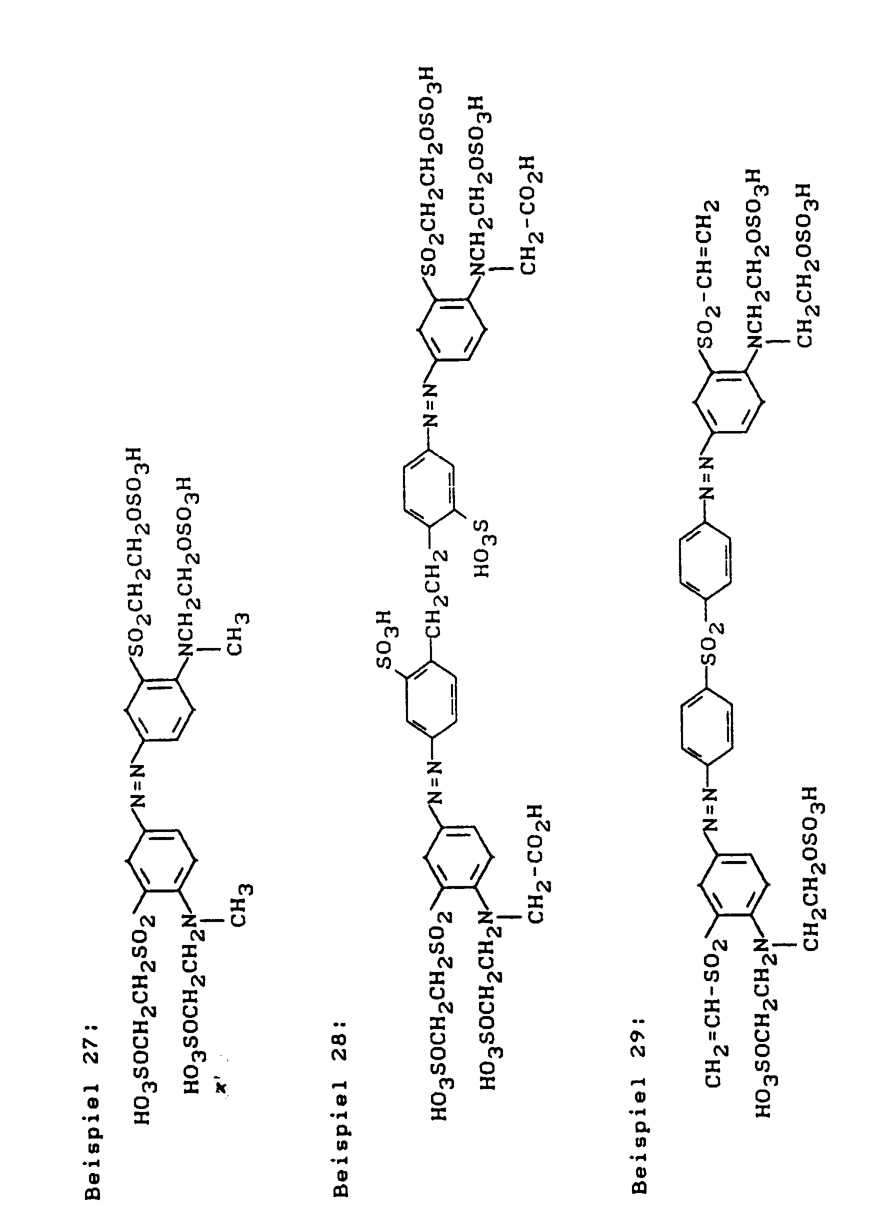

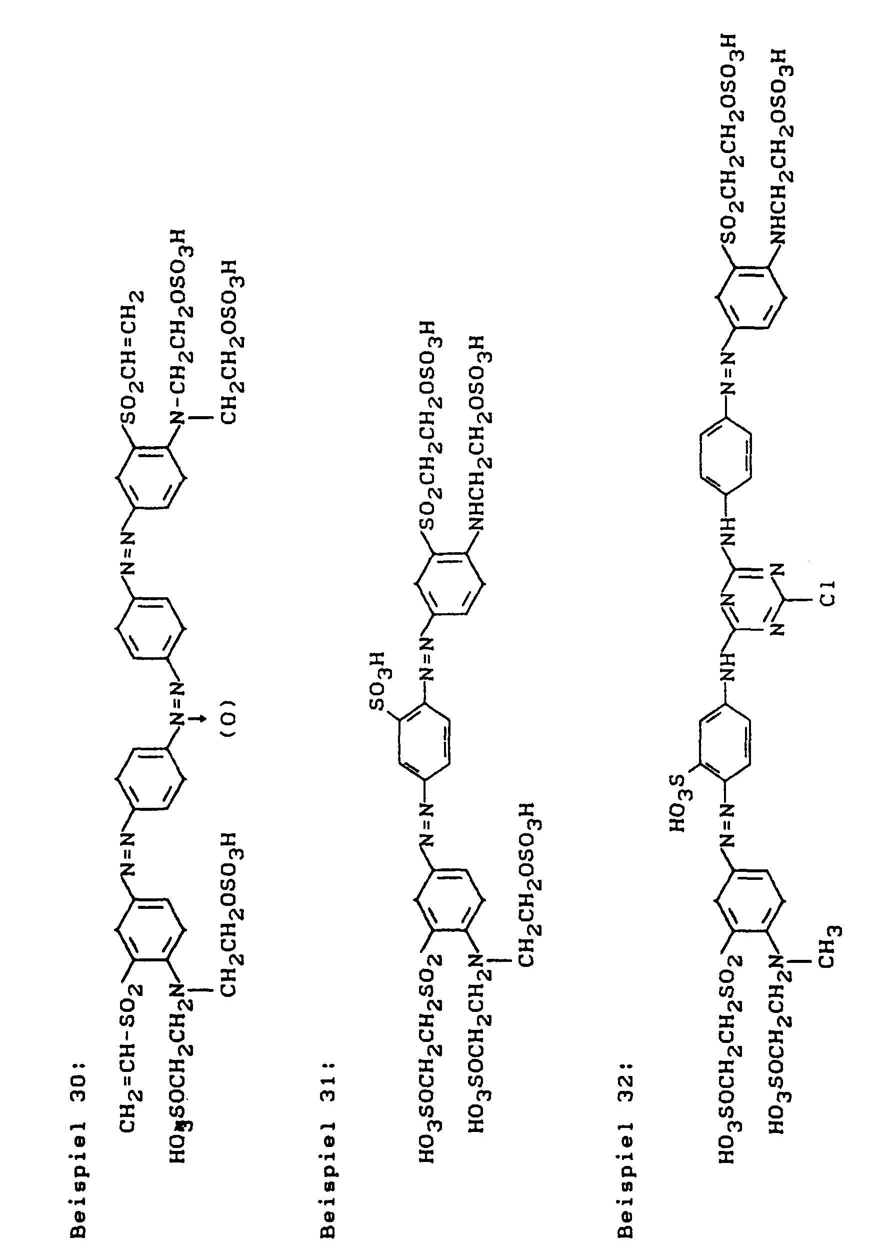

### Beispiel 33:

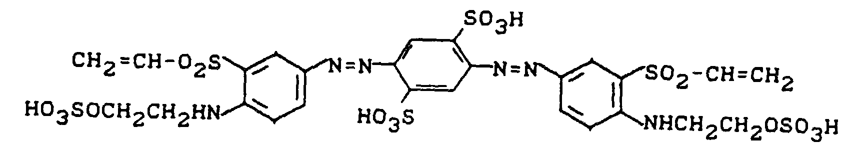

### Beispiel 34:

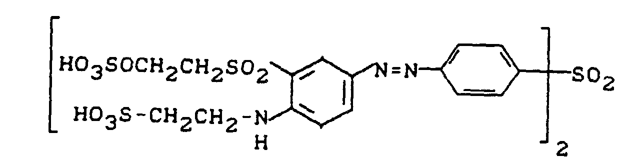

### Beispiel 35:

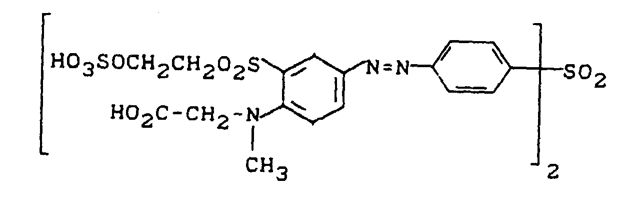

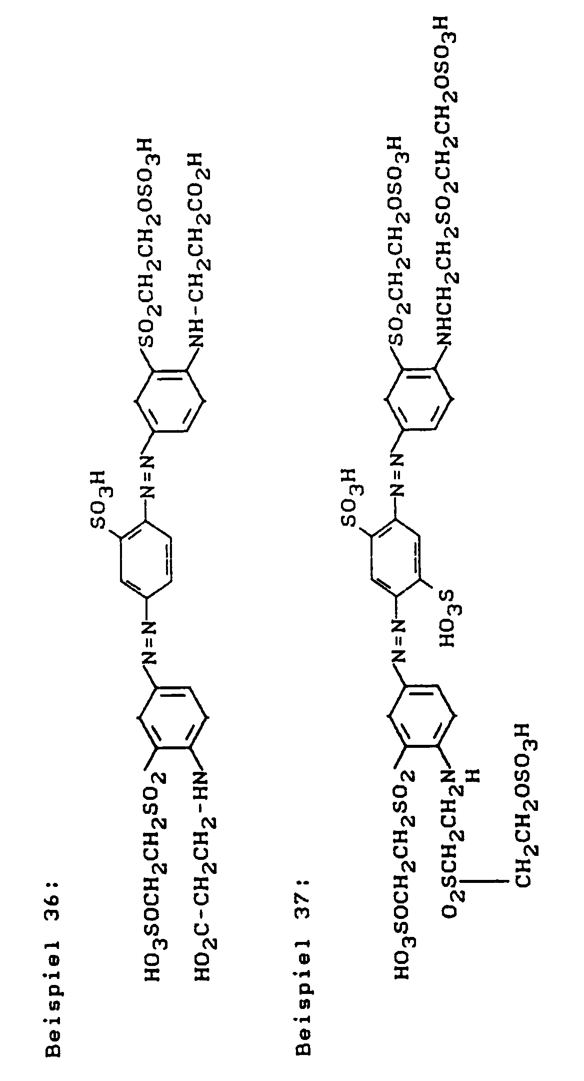

### Beispiel 38:

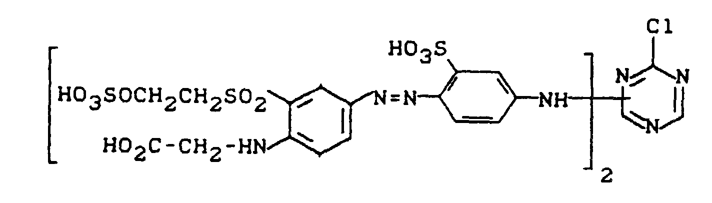

Variiert man in Beispiel 2 die Kupplungskomponente, so erhält man folgende wertvolle Reaktivfarbstoffe, die Baumwolle in klaren gelben Farbtönen färben:

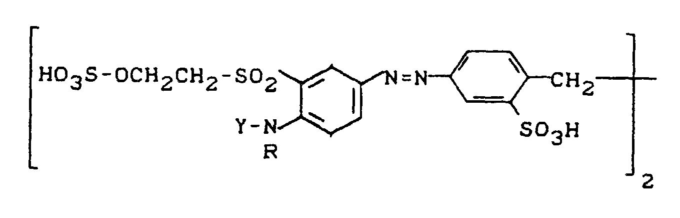

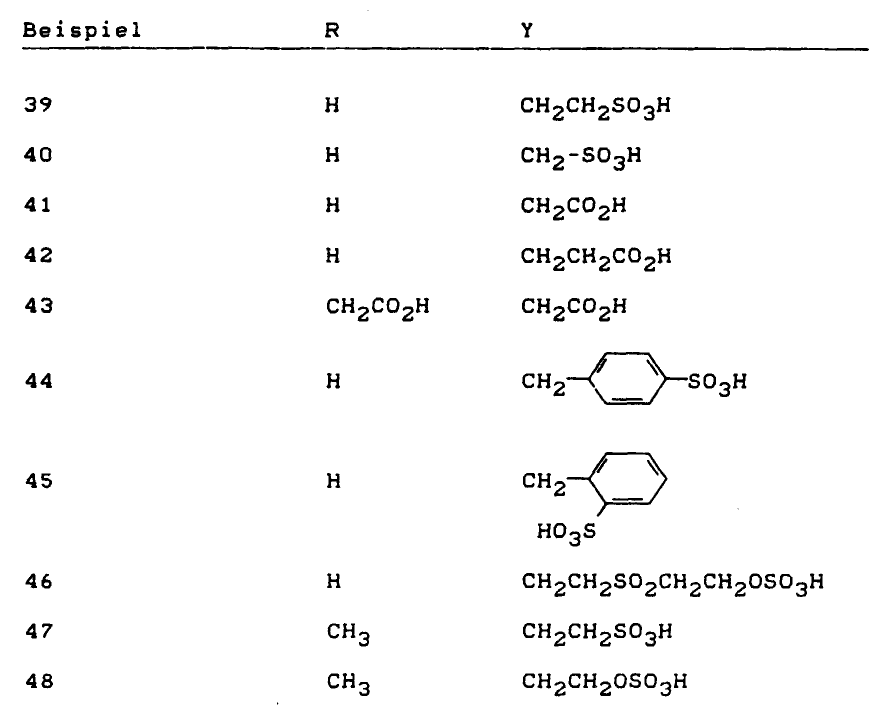

### Beispiel 49

Der Azofarbstoff der ersten Kupplung aus Beispiel 14, dem die für das erste Zwischenprodukt in Beispiel 17 beschriebene Struktur zukommt, wird als feuchte Paste in 500 ml Wasser bei 60°C gelöst. Mit ca. 35 ml konz. Natronlauge stellt man einen pH-Wert von 12,0 ein und fügt anschließend 20 g Traubenzucker zu. Die Reduktionsreaktion verläuft leicht exotherm. Es wird eine weitere Stunde bei 65-70°C gerührt, anschließend auf 20°C abgekühlt und auf pH 6,0 gestellt. Der ausgefallene Azo/Azoxy-Farbstoff, dem die Struktur (λₘₐₓ = 384, 467 nm (H₂O)) 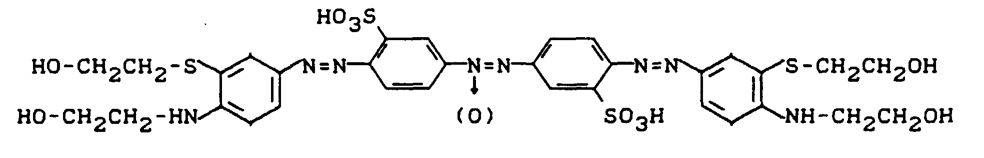 zukommt, wird abgesaugt und die isolierte Paste in 400 ml Wasser verrührt.

Nach Erwärmen auf 70°C und Zusatz von 0,2 g Natriumwolframat korrigiert man den pH-Wert auf 6,0 und tropft dann langsam 60 ml einer 35 %igen wäßrigen Wasserstoffperoxidlösung zu Die Reaktionstemperatur steigt dabei auf 80-85°C an. Der pH-Wert wird durch Sodazugabe bei 5,0 - 6,0 gehalten. Nach 45 Minuten ist die Oxidationsreaktion beendet.

Es wird auf 20°C abgekühlt, abgesaugt und das isolierte Produkt getrocknet. Es resultieren ca. 60 g salzhaltiges Produkt, dem die Struktur (λₘₐₓ = 451 nm (H₂O)) 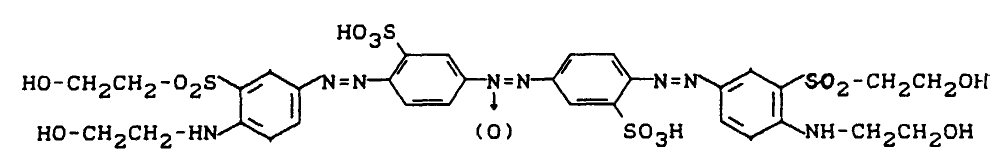 zukommt.

35 g dieses nichtfaserreaktiven Farbstoffs werden portionsweise bei 15-25°C in 100 ml Schwefelsäuremonohydrat eingetragen und bis zur Lösung verrührt. Bei 25-30°C versetzt man mit 20 ml 20 %igem Oleum und rührt eine Stunde nach. Diese Lösung trägt man anschließend auf eine Vorlage aus 100 ml gesättigter Kochsalzlösung und 300 g Eis aus. Der ausgefallene Farbstoff wird abgesaugt. Die isolierte saure Paste wird in 200 ml Wasser angerührt und mit festem Natriumhydrogencarbonat auf pH 5,5 eingestellt. Die Farbstofflösung wird am Rotationsverdampfer total aufkonzentriert und der resultierende, noch feuchte Farbstoff getrocknet. Man erhält ca. 55 g natriumsulfathaltigen Reaktivfarbstoff der Struktur (λₘₐₓ = 454 nm (H₂O)) 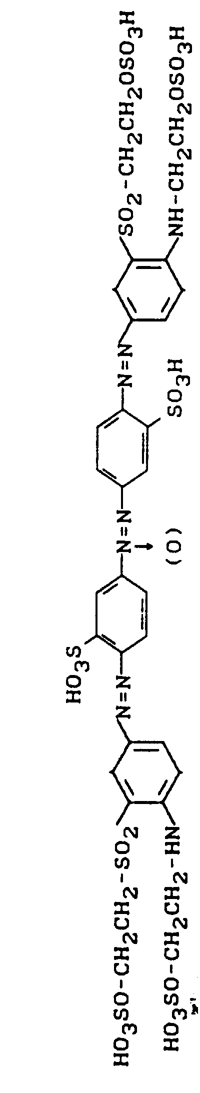 der Baumwolle in rotstichig braunen Tönen färbt.

Durch Variation von Diazo- bzw. Kupplungskomponente sind in Analogie zur Herstellungsvorschrift des Beispiels 49 auch Beispiel 30 sowie die nachfolgenden Farbstoffbeispiele zugänglich.

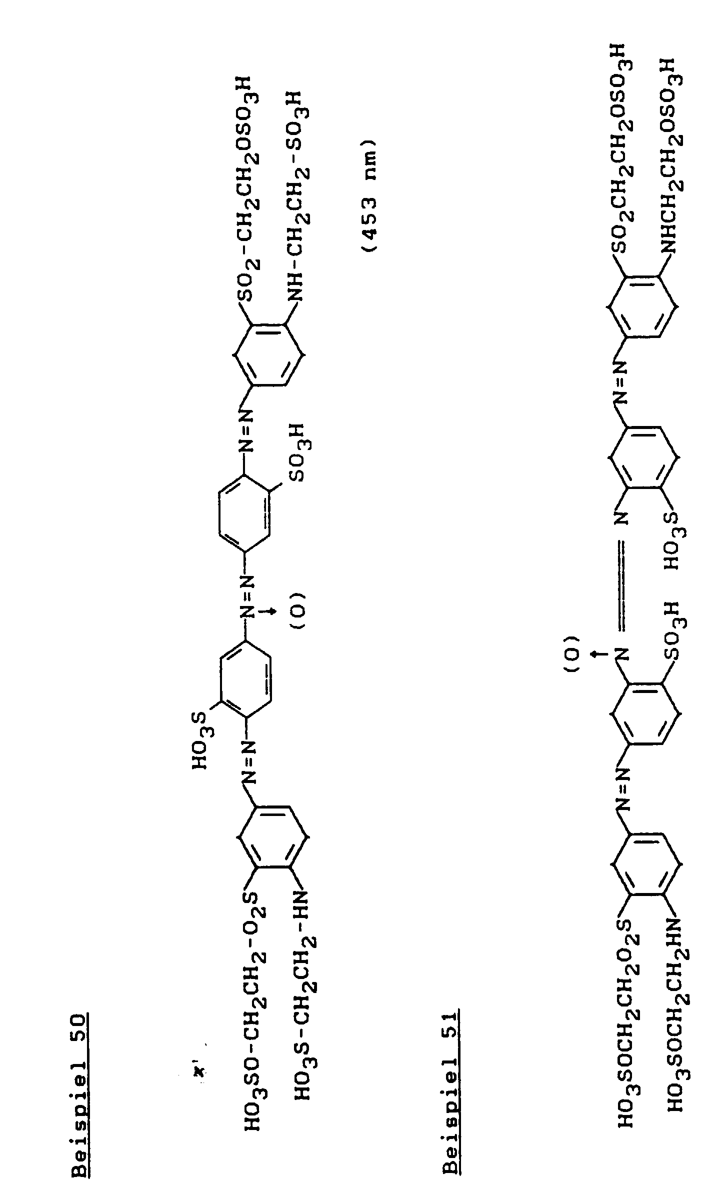

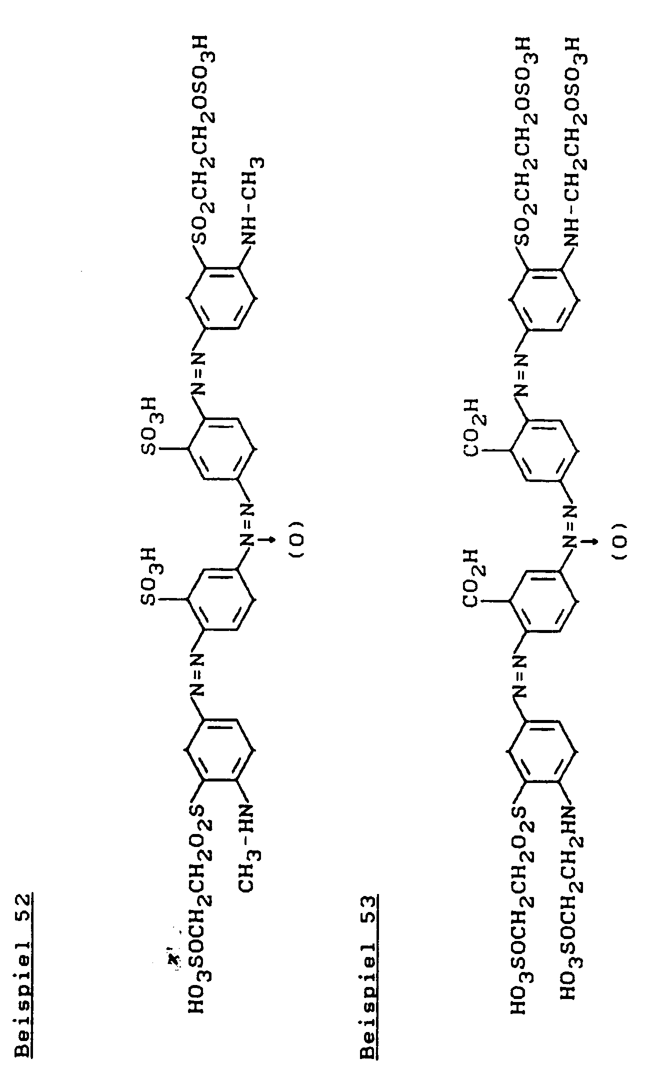

### Färbevorschrift 1

2 Teile des gemäß Beispiel 18 erhältlichen Farbstoffes werden in 100 ml Wasser gelöst. Die Lösung gibt man zu 1900 Teilen kaltem Wasser, fügt 60 Teile Natriumchlorid zu und geht mit 100 Teilen eines Baumwollgewebes in dieses Färbebad ein.

Man steigert die Temperatur auf 50°C, wobei nach 30 Minuten 40 Teile kalzinierte Soda und nochmals 60 Teile Natriumchlorid zugegeben werden. Man hält die Temperatur 30 Minuten auf 50°C, spült und seift dann die Färbung während 15 Minuten in einer 0,3%igen kochenden Lösung eines ionenfreien Waschmittels, spült und trocknet. Man erhält eine goldgelbe Färbung mit guten Echtheitseigenschaften.

### Färbevorschrift 2

4 Teile des in Beispiel 2 hergestellten Reaktivfarbstoffes werden in 50 Teilen Wasser gelöst. Dazu gibt man 50 Teile einer Lösung, die pro Liter 5 g Natriumhydroxyd und 10 g kalziniertes Soda enthält. Mit der erhaltenen Lösung wird ein Baumwollgewebe foulardiert, so daß es um 70 % seines Gewichtes zunimmt, und dann auf eine Kaule aufgewickelt. Das Baumwollgewebe wird so während 3 bis 12 Stunden bei Raumtemperatur gelagert. Danach wird die gefärbte Ware gespült, während eine Viertelstunde mit einem nichtionischen Waschmittel kochend geseift, nochmals gespült und getrocknet. Man erhält eine gelbe Färbung mit guten Echtheitseigenschaften. Tiefschwarze Färbungen werden erhalten, wenn man geeignete Mischungen von Farbstoffbeispiel 18 mit dem Farbstoff der Formel

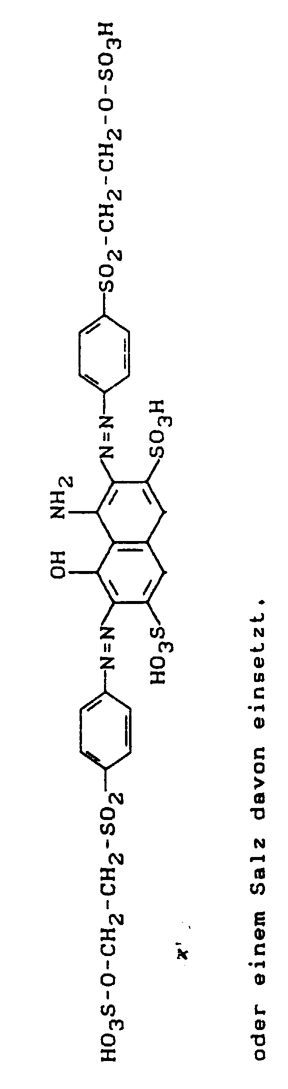

Grüne Färbungen resultieren, wenn man geeignete Mischungen von Farbstoffbeispiel 2 mit dem Farbstoff der Formel 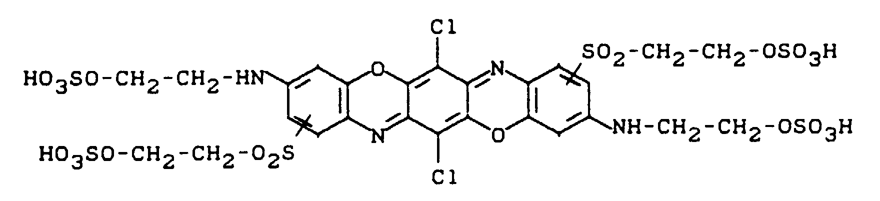 oder einem Salz davon einsetzt.

## Patentansprüche

1. Polyfunktionell faserreaktive Azofarbstoffe, die in Form der freien Säure der Formel 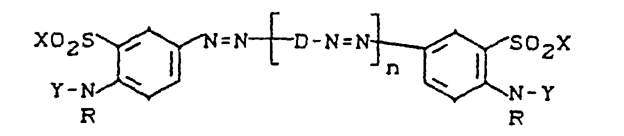 entsprechen,
worin
n = 0 oder 1,
X = CH=CH₂ oder CH₂CH₂OSO₃H,
Y = H, C₁-C₆-Alkyl, mit Cl, OH, CN, CO₂H, OSO₃H, SO₃H, SO₂X oder C₁-C₄-Alkoxy substituiertes C₁-C₆-Alkyl, Allyl, Cycloalkyl, mit CO₂H, OSO₃H oder SO₃H substituiertes Cycloalkyl, Benzyl oder mit OH, CO₂H oder SO₃H substituiertes Benzyl,
R = H, C₁-C₄-Alkyl, mit Cl, OH, CN, CO₂H, OSO₃H, SO₃H, SO₂X oder C₁-C₄-Alkoxy substituiertes C₁-C₄-Alkyl, Allyl, Benzyl oder mit OH, CO₂H bzw. Sulfo substituiertes Benzyl,
wobei NR-Y einen gesättigten Heterocyclus darstellen kann,
D = ein bivalenter aromatischer oder heteroaromatischer Rest oder Formel 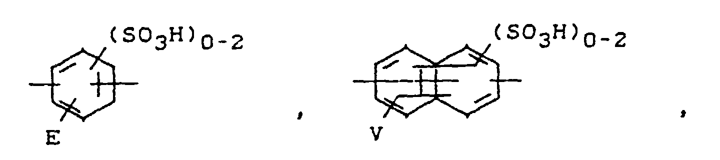 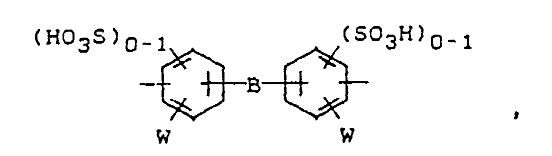 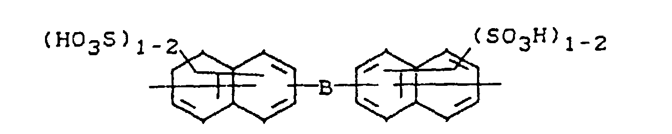 oder 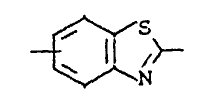 mit
E, V, W = unabhängig voneinander Wasserstoff oder C₁-C₄-Alkyl, C₁-C₄-Alkoxy, Halogen oder Carboxy.
B = direkte Bindung oder ein Brückenglied.

2. Farbstoffe gemäß Anspruch 1, worin
n = 1.

3. Farbstoffe gemäß wenigstens einem der vorhergehenden Ansprüche, dadurch gekennzeichnet, daß
B = CH₂, CH₂-CH₂, CH=CH, C≡C, O, S, SO, SO₂, CO, CO₂, OCH₂CH₂O, CH₂-O-CH₂, CH₂CH₂BCH₂CH₂, NHCO, NHCONH, NR¹, N=N, N=N→O, O←N=N→O, NHCOCONH, 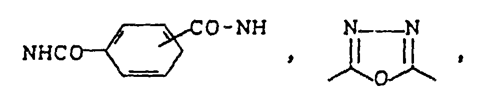 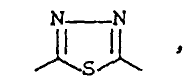 oder ein Triazinylrest der Struktur 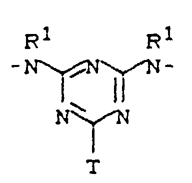 worin
R¹ = jeweils unabhängig voneinander H oder C₁-C₄-Alkyl und
T = F, Cl, Br, OR², SR², NR³R⁴,
R² = H, C₁-C₆-Alkyl, mit OH, Halogen, C₁-C₄-Alkoxy, CO₂H, SO₃H bzw. OSO₃H substituiertes C₁-C₆-Alkyl, Cyclohexyl, Furfuryl, Phenyl, mit OH, CO₂H, SO₃H, C₁-C₄-Alkyl oder C₁-C₄-Alkoxy substituiertes Phenyl oder mit OH, CO₂H oder SO₃H substituiertes oder unsubstituiertes Naphthyl,
R³, R⁴ = unabhängig voneinander Wasserstoff, C₁-C₆-Alkyl, C₁-C₄-Alkenyl, mit Halogen, OH, CO₂H, SO₃H, OSO₃H, Methoxy, Ethoxy, SO₂Z oder OCH₂CH₂SO₂Z substituiertes C₁-C₆-Alkyl, Cyclohexyl, Cyclopentyl, Benzyl, Phenyl beziehungsweise Naphthyl sowie mit C₁-C₄-Alkyl, C₁-C₄-Alkoxy, Halogen, CO₂H, SO₃H, SO₂Z oder CH₂SO₂Z substituiertes Phenyl, Benzyl beziehungsweise Naphthyl, wobei R³ und R⁴ zusammen mit dem N-Atom den Rest eines 5- oder 6-gliedrigen Heterocyclus bilden können,
Z = CH=CH₂, CH₂CH₂OSO₃H, CH₂CH₂Cl.

4. Reaktivfarbstoffe gemäß einem der vorhergehenden Ansprüche, dadurch gekennzeichnet, daß sie einer der folgenden Formeln entsprechen
A-N=N-A 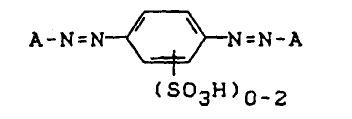 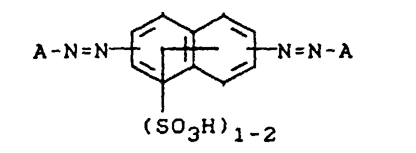 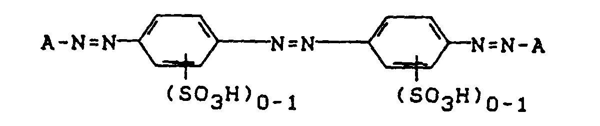 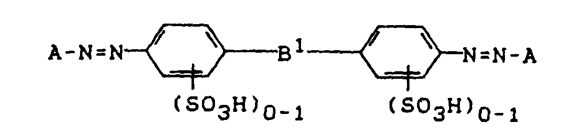
mit 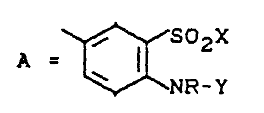 und
B¹ = direkte Bindung oder CH₂CF₂, CH=CH, CO, SO₂, NHCONH sowie ein Triazinylrest der Struktur 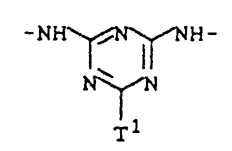
worin
T¹ = Cl, F, OH oder NR³R⁴,
X = CH=CH₂, C₂H₄OSO₃H und wobei
R, Y, R³ und R⁴ die in Anspruch 1 angegebene Bedeutung haben.

5. Verbindung der Formel 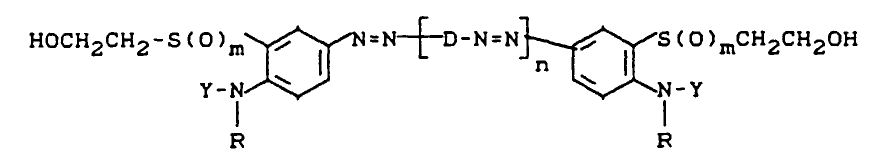 worin
m = unabhängig voneinander 0 oder 2,
n = 1 und
R, Y und D die in Anspruch 1 genannte Bedeutung zukommt.

6. Verbindung der Formel 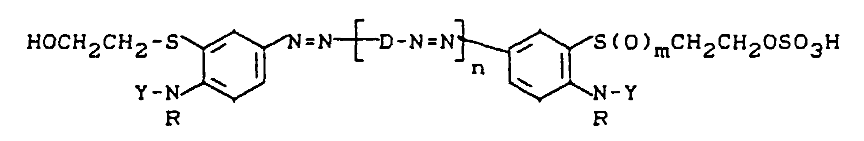 worin
m = 0 oder 2,
n = 0 oder 1 und
R, Y und D die in Anspruch 1 genannte Bedeutung zukommt.

7. Verbindung der Formel 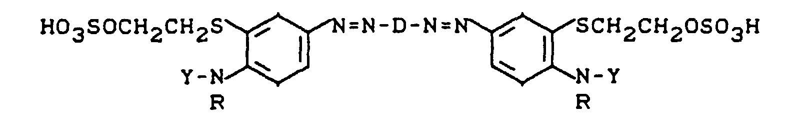 worin
R, Y und D die in Anspruch 1 genannte Bedeutung zukommt.

8. Verfahren zum Färben oder Bedrucken von hydroxyl- oder amidgruppenhaltigen Materialien mit einem Reaktivfarbstoff, dadurch gekennzeichnet, daß ein Reaktivfarbstoff gemäß Anspruch 1 verwendet wird.

## Claims

1. Polyfunctional fibre-reactive azo dyestuffs which, in the form of the free acid, correspond to the formula 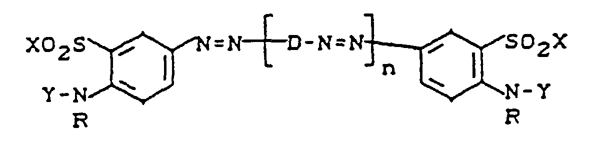 wherein
n = 0 or 1,
X = CH=CH₂ or CH₂CH₂OSO₃H,
Y = H, C₁-C₆-alkyl, C₁-C₆-alkyl which is substituted by Cl, OH, CN, CO₂H, OSO₃H, SO₃H, SO₂X or C₁-C₄-alkoxy, allyl, cycloalkyl, cycloalkyl which is substituted by CO₂H, OSO₃H or SO₃H, benzyl or benzyl which is substituted by OH, CO₂H or SO₃H,
R = H, C₁-C₄-alkyl, C₁-C₄-alkyl which is substituted by Cl, OH, CN, CO₂H, OSO₃H, SO₃H, SO₂X or C₁-C₄-alkoxy, allyl, benzyl or benzyl which is substituted by OH, CO₂H or sulpho,
wherein NR-Y can represent a saturated heterocyclic ring, and
D = a bivalent aromatic or heteroaromatic radical of the formula 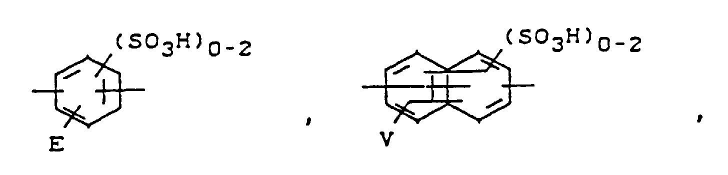 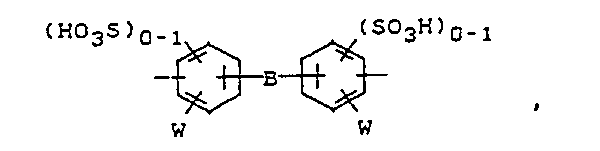 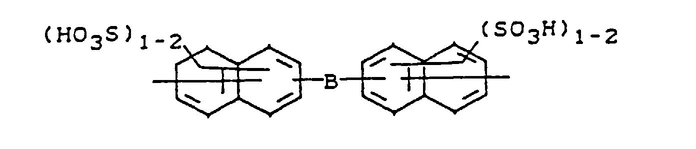 or 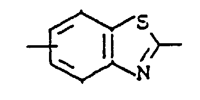
where
E, V and W = independently of one another, hydrogen or C₁-C₄-alkyl, C₁-C₄-alkoxy, halogen or carboxyl.
B = a direct bond or a bridge member.

2. Dyestuffs according to Claim 1, wherein
n = 1.

3. Dyestuffs according to at least one of the preceding claims, characterised in that
B = CH₂, CH₂-CH₂, CH=CH, O, S, SO, SO₂, CO, CO₂, OCH₂CH₂O, CH₂-O-CH₂, CH₂CH₂OCH₂CH₂, NHCO, NHCONH, NR¹, N=N, N=N→O, O←N=N→O, NHCOCONH, 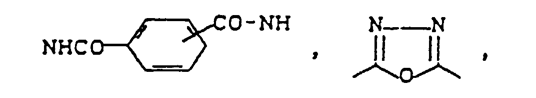 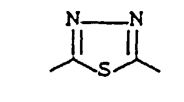 or a triazinyl radical having the structure 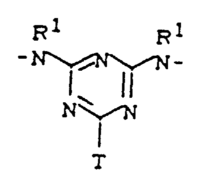 wherein
R¹ = in each case independently of one another, H or C₁-C₄-alkyl and
T = F, Cl, Br, OR², SR² or NR³R⁴,
R² = H, C₁-C₆-alkyl, C₁-C₆-alkyl which is substituted by OH, halogen, C₁-C₄-alkoxy, CO₂H, SO₃H or OSO₃H, cyclohexyl, furfuryl, phenyl, phenyl which is substituted by OH, CO₂H, SO₃H, C₁-C₄-alkyl or C₁-C₄-alkoxy, or naphthyl which is substituted by OH, CO₂H or SO₃H or is unsubstituted,
R³ and R⁴ = independently of one another, hydrogen, C₁-C₆-alkyl, C₁-C₄-alkenyl, C₁-C₆-alkyl which is substituted by halogen, OH, CO₂H, SO₃H, OSO₃H, methoxy, ethoxy, SO₂Z or OCH₂CH₂SO₂Z, cyclohexyl, cyclopentyl, benzyl, phenyl or naphthyl, or phenyl, benzyl or naphthyl which is substituted by C₁-C₄-alkyl, C₁-C₄-alkoxy, halogen, CO₂H, SO₃H, SO₂Z or CH₂SO₂Z, and wherein R³ and R⁴, together with the N atom, can form the radical of a 5- or 6-membered heterocyclic ring,
Z = CH=CH₂, CH₂CH₂OSO₃H or CH₂CH₂Cl.

4. Reactive dyestuffs according to one of the preceding claims, characterised in that they correspond to one of the following formulae
A-N=N-A 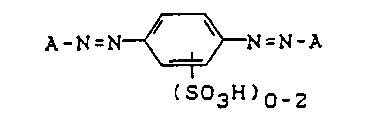 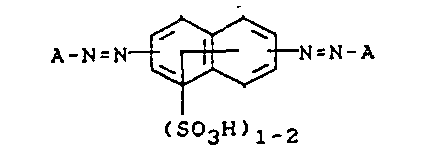 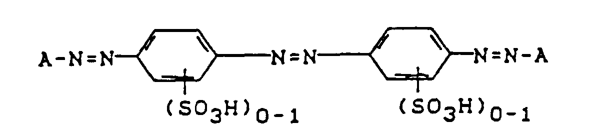 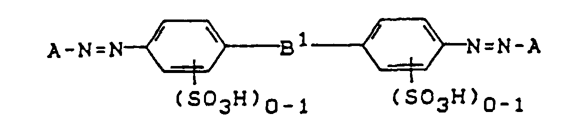
where A = 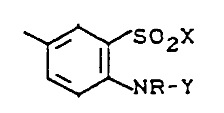 and
B¹ = a direct bond or CH₂CH₂, CH=CH, CO, SO₂, NHCONH or a triazinyl radical having the structure 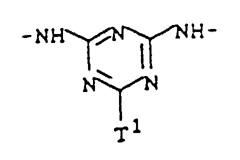
wherein
T¹ = Cl, F, OH or NR³R⁴, and
X = CH=CH₂ or C₂H₄OSO₃H, and wherein
R, Y, R³ and R⁴ have the meaning given in Claim 1.

5. Compound of the formula 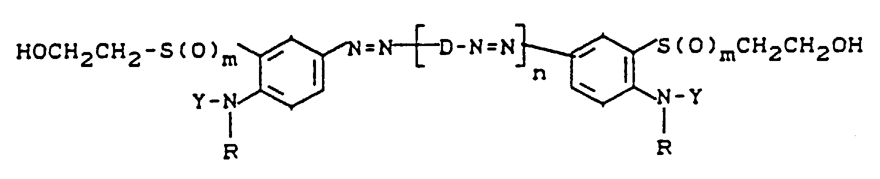 wherein
m = independently of one another, 0 or 2,
n = 1 and
R, Y and D have the meaning given in Claim 1.

6. Compound of the formula 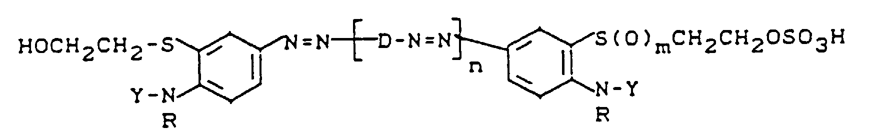 wherein
m = 0 or 2,
n = 0 or 1 and
R, Y and D have the meaning given in Claim 1.

7. Compound of the formula 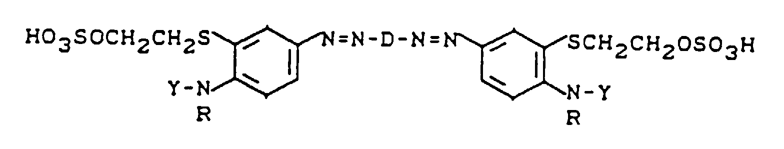 wherein
R, Y and D have the meaning given in Claim 1.

8. Process for dyeing or printing materials containing hydroxyl groups or amide groups with a reactive dyestuff, characterised in that a reactive dyestuff according to Claim 1 is used.

## Revendications

1. Colorants azoïques polyfonctionnels réactifs avec les fibres qui, à l'état d'acides libres, répondent à la formule 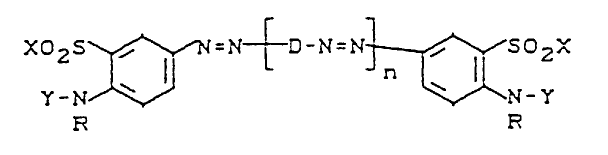 dans laquelle n = 0 ou 1,
X représente CH=CH₂ ou CH₂CH₂OSO₃H,
Y représente H, un groupe alkyle en C₁ à C₆, un groupe alkyle en C₁ à C₆ substitué par Cl, OH, CN, CO₂H, OSO₃H, SO₃H, SO₂X ou alcoxy en C₁ à C₄, un groupe allyle, un groupe cycloalkyle, un groupe cycloalkyle substitué par CO₂H, OSO₃H ou SO₃H, un groupe benzyle ou un groupe benzyle substitué par OH, CO₂H ou SO₃H,
R représente H, un groupe alkyle en C₁ à C₄, un groupe alkyle en C₁ à C₄ portant des substituants Cl, OH, CN, CO₂H, OSO₃H, SO₃H, SO₂X ou alcoxy en C₁ à C₄, un groupe allyle, benzyle ou benzyle portant des substituants OH, CO₂H ou sulfo,
NR-Y pouvant également représenter un hétérocycle saturé,
D représente un radical aromatique ou hétéroaromatique bivalent de formule 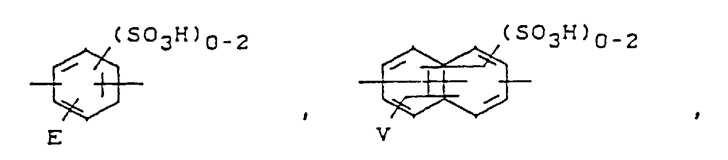 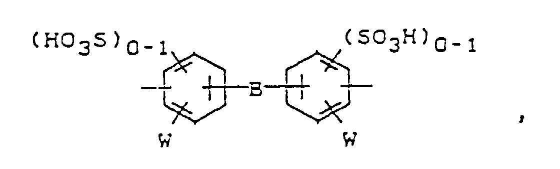 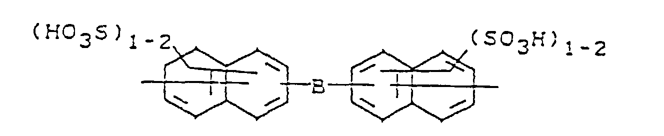 ou 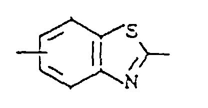
dans lesquelles
E, V, W représentent chacun, indépendamment les uns des autres, l'hydrogène ou un groupe alkyle en C₁ à C₄, alcoxy en C₁ à C₄, un halogène ou un groupe carboxy et
B représente une liaison directe ou un pont.

2. Colorants selon revendication 1, pour lesquels n = 1.

3. Colorants selon au moins une des revendications qui précèdent, caractérisés en ce que
B représente une liaison directe ou un pont, par exemple CH₂, CH₂-CH₂, CH=CH, C≡C, O, S, SO, SO₂, CO, CO₂, OCH₂CH₂O, CH₂-O-CH₂, CH₂CH₂OCH₂CH₂, NHCO, NHCONH, NR¹, N=N, N=N→O, O←N=N→O, NHCOCONH, 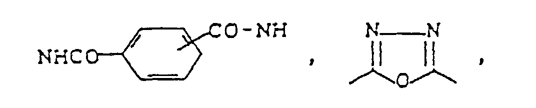 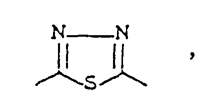 ou un groupe triazinyle de structure 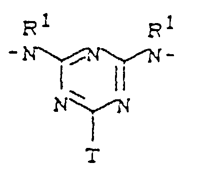
dans laquelle
les symboles R¹ représentent chacun, indépendamment l'un de l'autre, H ou un groupe alkyle en C₁ à C₄ et
T représente F, Cl, Br, OR², SR², NR³R⁴,
R² représente H, un groupe alkyle en C₁ à C₆, un groupe alkyle en C₁ à C₆ portant des substituants OH, halogéno, alcoxy en C₁ à C₄, CO₂H, SO₃H ou OSO₃H, un groupe cyclohexyle, furfuryle, phényle, phényle portant des substituants OH, CO₂H, SO₃H, alkyle en C₁ à C₄ ou alcoxy en C₁ à C₄, ou naphtyle non substitué ou portant des substituants OH, CO₂H ou SO₃H,
R³ et R⁴ représentent chacun, indépendamment l'un de l'autre, l'hydrogène, un groupe alkyle en C₁ à C₆, alcényle en C₁ à C₄, un groupe alkyle en C₁ à C₆ portant des substituants halogéno, OH, CO₂H, SO₃H, OSO₃H, méthoxy, éthoxy, SO₂Z ou OCH₂CH₂SO₂Z, un groupe cyclohexyle, cyclopentyle, benzyle, phényle ou naphtyle, ou bien un groupe phényle, benzyle ou naphtyle portant des substituants alkyle en C₁ à C₄, alcoxy en C₁ à C₄, halogéno, CO₂H, SO₃H, SO₂Z ou CH₂SO₂Z, R³ et R⁴ pouvant également former ensemble et avec l'atome d'azote le radical d'un hétérocycle à 5 ou 6 chaînons, et
Z représente CH=CH₂, CH₂CH₂OSO₃H, CH₂CH₂Cl.

4. Colorants réactifs selon l'une des revendications qui précédent, caractérisés en ce qu'ils répondent à l'une des formules suivantes :
A-N=N-A 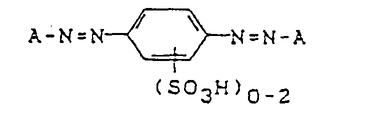 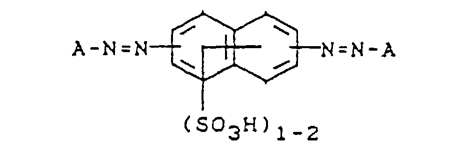 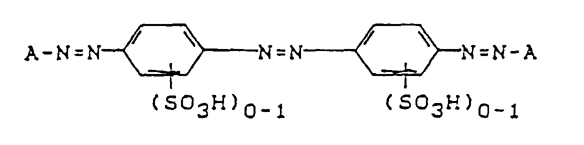 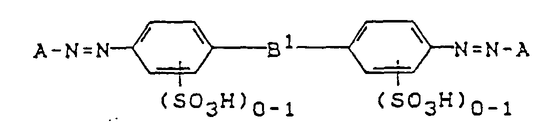
dans lesquelles A = 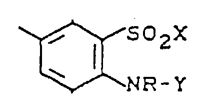 et
B¹ représente une liaison directe ou CH₂CH₂, CH=CH, CO, SO₂, NHCONH ou un groupe triazinyle de structure 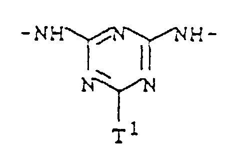
dans laquelle
T¹ = Cl, F, OH ou NR³R⁴,
X = CH=CH₂ ou C₂H₄OSO₃H et
R, Y, R³ et R⁴ ayant les significations indiquées dans la revendication 1.

5. Composé de formule 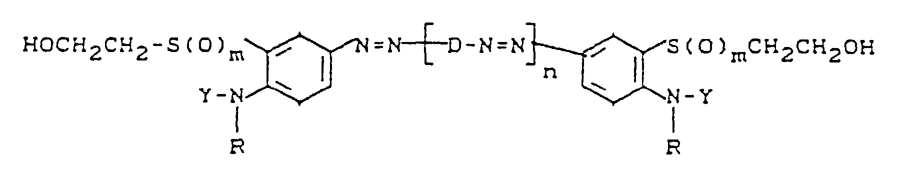 dans laquelle
les indices m sont égaux chacun, indépendamment l'un de l'autre, à 0 ou 2,
n = 1 et
R, Y et D ont les significations indiquées dans la revendication 1.

6. Composé de formule 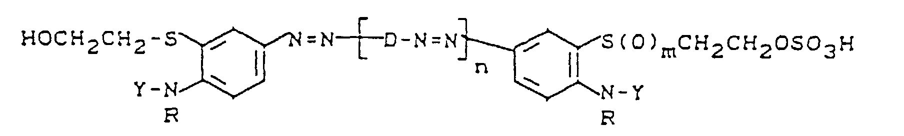 dans laquelle
m = 0 ou 2,
n = 0 ou 1 et
R, Y et D ont les significations indiquées dans la revendication 1.

7. Composé de formule 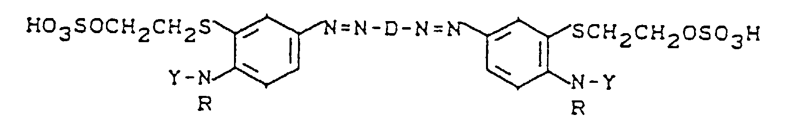 dans laquelle
R, Y et D ont les significations indiquées dans la revendication 1.

8. Procédé pour la teinture ou l'impression de matières contenant des groupes hydroxy ou des groupes amides à l'aide d'un colorant réactif, caractérisé en ce que l'on utilise un colorant réactif selon revendication 1.
